# EUROPEAN PATENT APPLICATION

(11) **EP 2 510 949 A1**
(43) Date of publication of application: **17.10.2012**
(21) Application number: 10836055.3
(22) Date of filing: 10.12.2010
(51) Int. Cl.: A61K 45/00, A61K 31/137, A61K 31/335, A61K 31/343, A61K 31/353, A61K 31/397, A61K 31/4025, A61K 31/4035, A61K 31/407, A61K 31/415

(54) **THERAPEUTIC AGENT FOR FIBROMYALGIA**

(30) Priority: 11.12.2009 JP 2009281490
(71) Applicant: Astellas Pharma Inc., Tokyo 103-8411 (JP)
(72) Inventor: NAGAKURA, Yukinori, Tokyo 103-8411 (JP); TSUKAMOTO, Mina, Tokyo 103-8411 (JP); WATABIKI, Tomonari, Tokyo 103-8411 (JP)
(74) Representative: HOFFMANN EITLE
(86) International application number: PCT/JP2010/072194
(87) International publication number: WO 2011/071136

(57) **Abstract**

An agent for treating fibromyalgia containing a 5-HT_{2C} receptor agonist as an active ingredient

## Description

### Technical Field

The present invention relates to a drug, particularly, to an agent for treating fibromyalgia.

### Background Art

Fibromyalgia is a chronic pain disease that induces intolerable chronic pain throughout the whole body as a main symptom accompanied by various concomitant symptoms such as insomnia, generalized fatigue, and depression. Symptoms of fibromyalgia are very diverse. The pain symptoms of fibromyalgia are characterized by being accompanied by chronic pain of deep tissues such as muscle tissues and pain caused by finger pressure. In many cases, the symptoms are also accompanied by pain called tactile allodynia or cold allodynia caused by a tactile stimulus or a cold stimulus that does not in general cause pain. In addition, the symptoms are accompanied by thermal hyperalgesia caused by hypersensitivity to a thermal stimulus in many cases. A higher proportion of patients with fibromyalgia suffer from the concomitant symptoms such as emotional disturbance like depression and anxiety, fatigue, sleep disturbance, and irritable bowel syndrome compared to patients with other pain diseases (such as peripheral neuropathy, post-herpetic pain, complex regional pain syndrome, diabetic neuropathy, cancer pain, rheumatism, osteoarthritis, acute postoperative pain, and the like). While organic and functional disorders as the cause of the pain are clear in other pain diseases, the causes of the pain are not clearly explicable for patients with fibromyalgia.
According to the diagnosis criterion defined by the American College of Rheumatology, if pain continues for 3 months or longer over a wide area of the body, and tenderness is perceived in 11 or more sites among tender points of 18 sites throughout the whole body (ligaments close to bones, tendons, muscles, and the like), it is diagnosed as fibromyalgia. This diagnosis criterion is clearly distinguished from the diagnosis criterion of other pain diseases. That is, fibromyalgia is an independent chronic disease that is clearly distinguished from other pain diseases from the viewpoints of symptoms, the cause of pain, the diagnosis criterion, and the like.

The prevalence rate of fibromyalgia accounts for about 2% of the population, so there are a great number of patients. However, since there is no sufficiently effective therapeutic method, the development of a highly effective new therapeutic method is strongly desired.

The pathological mechanism of fibromyalgia has not been sufficiently clarified. The intolerable chronic pain of the whole body that is a characteristic of fibromyalgia and various concomitant symptoms such as insomnia, generalized fatigue, and depression cannot be sufficiently explained from the disorders at the peripheral level. Consequently, it is considered that a disorder in a pain control system in the central nervous system is involved in the pathological mechanism of fibromyalgia (for example, see Non-Patent Document 1). Actually, results of research using functional magnetic resonance imaging (fMRI) or single-photon emission tomography (SPET) shows that there are abnormalities in the brain function of patients with fibromyalgia (for example, see Non-Patent Documents 2 and 3).

In patients with fibromyalgia, quantitative fluctuation and functional abnormalities of various neurotransmitters, cytokines, or hormones are observed. The concentration of excitatory amino acids, substance P, or nerve growth factor in the cerebrospinal fluid of patients with fibromyalgia is higher than that of a non-patient group. On the other hand, the concentration of the metabolites of serotonin, dopamine, or norepinephrine in the cerebrospinal fluid of patients with fibromyalgia is lower than that of a non-patient group (for example, see Non-Patent Document 4). There is a report reporting that the amount of excitatory amino acid which is an brain excitatory transmitter in the insula of the brain is correlated with the pain level of patients with fibromyalgia (for example, see Non-Patent Document 5).
In addition, there is a report reporting that patients with fibromyalgia show an abnormality in a brain mechanism of releasing dopamine in response to a pain stimulation (for example, see Non-Patent Document 4). From the skin tissue of patients with fibromyalgia, cytokines such as interleukin-1 or a tumor necrosis factor are detected (for example, see Non-Patent Document 6). Moreover, it is suggested that the function of secreting growth hormone or insulin-like growth factor is depressed in patients with fibromyalgia (for example, see Non-Patent Document 4). As described so far, a possibility that many neurotransmitters, cytokines, hormones, and the like may be involved in the pathological mechanism of fibromyalgia has been suggested. However, at this point in time, it is unclear what type of change is the cause of onset and which is the concomitant phenomenon. In other words, the pathological mechanism of fibromyalgia has not been clarified (for example, see Non-Patent Document 6).

In recent years, there has been a report reporting that pregabalin as a neuronal Ca²⁺ channel ligand, duloxetine as a selective serotonin-norepinephrine reuptake inhibitor, or pramipexole as a dopamine receptor agonist statistically significantly reduces the score of pain symptoms of patients with fibromyalgia, compared to a placebo group (for example, see Non-Patent Documents 7 to 9).
However, the effect of these drugs is limited, and a therapeutic method that is sufficiently effective for the symptoms of patients with fibromyalgia including pain as a main symptom has not been found so far. Therefore, the development of a superior therapeutic agent having sufficient effects and few side effects is strongly required.

In order to create an agent for treating fibromyalgia, disease animal models which make it possible to sufficiently reflect disease conditions of fibromyalgia from the viewpoints of face validity, construct validity, and predictive validity and to efficiently screen test substances are indispensable. The present inventors administered a drug (for example, reserpine) reducing biogenic amine level in the body to a mammal so as to continuously induce chronic pain symptoms (myalgia and tactile allodynia) and depression which are characteristic symptoms of fibromyalgia and developed animal models of fibromyalgia by which clinical effects of a therapeutic agent is excellently predicted (for example, see Non-Patent Document 10).

A 5-HT_{2C} receptor is one of 14 serotonin receptor subtypes which exist at least in this number and is a G protein-coupled receptor. While almost not being expressed in peripheral tissues, the 5-HT_{2C} receptor is expressed in the central nervous system over a wide range. In the central nervous system, the 5-HT_{2C} receptor is distributed in the choroid plexus, prefrontal cortex, anterior olfactory nucleus, hippocampus, amygdala, basal ganglia, nucleus accumbens, ventral tegmental area, hypothalamus, and the like. From the distribution pattern expressed in the central nervous system, analysis results of knockout mouse of the 5-HT_{2C} receptor, and the like, a possibility that the 5-HT_{2C} receptor may be involved in various disease conditions such as obesity, metabolism disorders, epilepsy, anxiety, sleep disorders, drug dependence, dyskinesia, obsessive-compulsive disorder, schizophrenia, and depression has been suggested (for example, see Non-Patent Documents 11 and 12).

Since there is a suggestion that the 5-HT_{2C} receptor may be involved in various pathological conditions, there is a possibility that a 5-HT_{2C} receptor agonist may be useful as a therapeutic or prophylactic agent for various diseases. A large number of 5-HT_{2C} receptor agonists are known (for example, see Non-Patent Documents 11, 12, and 13). Examples of diseases likely to be applicable include central nervous system disorders (for example, depression, atypical depression, bipolar disorder, anxiety disorders, obsessive-compulsive disorder, social phobia, panic disorder, sleep disturbance, sexual dysfunction, schizophrenia, migraine, headache, other conditions accompanying other pains, intracranial hypertension, epilepsy, personality disorders, age-related behavioral abnormalities, behavior disorder accompanied by dementia, organic mental disorders, mental disorder in infancy, agitation, age-related memory disorder, chronic fatigue syndrome, drug addiction, alcoholism, drug dependence, obesity, hyperphagia, anorexia nervosa, premenstrual tension; damage to the central nervous system); cardiovascular disorders (for example, thrombosis); gastrointestinal disorders (for example, gastrointestinal motility insufficiency); uremia; sleep apnea; obesity; ocular hypertension; diabetes, and the like (for example, see Patent Documents 1 to 6 and the like, or Non-Patent Documents 11 and 12). In addition, there is a report regarding a therapeutic effect on urinary incontinence (for example, see Patent Documents 7 and 8).

Serotonin which is an intrinsic agonist of the 5-HT_{2C} receptor is considered to play an important role in an endogenous pain control mechanism in the spine. There is a possibility that exogenous administration of the 5-HT_{2C} receptor agonist may show an analgesic action by activating the endogenous pain control mechanism. Hitherto, it has been found that the exogenous administration of the 5-HT_{2C} receptor agonist shows analgesic action in an animal model of inflammatory pain (formalin-administered model or the like) or in an animal model of neuropathic pain (spinal nerve-ligated model or the like) (for example, see Non-Patent Documents 14 and 15). Meanwhile, there have been no disease animal models which make it possible to sufficiently reflect disease conditions of fibromyalgia and to efficiently screen test substances, until the present inventors administered a drug (for example, reserpine) reducing biogenic amine level in the body to a mammal so as to continuously induce chronic pain symptoms (myalgia and tactile allodynia) and depression which are characteristic symptoms of fibromyalgia and developed animal models of fibromyalgia by which clinical effects of a therapeutic agent is excellently predicted (for example, see Non-Patent Document 10). Consequently, it had been very difficult to verify the therapeutic effect of the 5-HT_{2C} receptor agonist on fibromyalgia, until the present inventors developed the animal model of fibromyalgia. In other words, the therapeutic effect of the 5-HT_{2C} receptor agonist on fibromyalgia was unclarified.

### Related Art

### Patent Document

[Patent Document 1] WO 98/56768
[Patent Document 2] WO 01/83487
[Patent Document 3] WO 03/86306
[Patent Document 4] WO 03/91250
[Patent Document 5] WO 03/33497
[Patent Document 6] WO 06/19886
[Patent Document 7] WO 04/96196
[Patent Document 8] WO 06/22420

### Non-Patent Document

[Non-Patent Document 1] Nature Clinical Practice Rheumatology, 2006, vol. 2, pp. 90-97
[Non-Patent Document 2] Journal of Rheumatology, 2004, vol. 31, pp. 364-378
[Non-Patent Document 3] American Journal of the Medical Sciences, 1998, vol. 315, pp. 385-396
[Non-Patent Document 4] Current Opinion in Investigational Drugs, 2007, vol. 16, pp.829-841
[Non-Patent Document 5] Arthritis & Rheumatism, 2008, vol. 58, pp.903-907
[Non-Patent Document 6] Joint Bone Spine, 2008, vol. 75, pp.273-279
[Non-Patent Document 7] The Journal of Rheumatology, 2008, vol. 35, pp.502-514
[Non-Patent Document 8] Pain, 2008, vol. 136, pp.432-444
[Non-Patent Document 9] Arthritis & Rheumatism, 2005, vol. 52, pp.2495-2505
[Non-Patent Document 10] Pain, 2009, vol. 146, No. 1-2, pp.26-33
[Non-Patent Document 11] Pharmacology & Therapeutics, 2008, vol. 119, No. 1, pp.7-23
[Non-Patent Document 12] Current opinion in drug discovery & development, 2008, vol. 11, No. 4, pp.43 8-445
[Non-Patent Document 13] Current Topics in Medical Chemistry, 2006, vol. 6, pp.1927-1970
[Non-Patent Document 14] Anesthesia & Analgesia, 2003, vol. 96, No. 4, pp. 1072-1078
[Non-Patent Document 15] Pain, 2004, vol. 108, No. 1-2, pp.163-169

### Disclosure of Invention

### Problems to Be Solved by the Invention

In order to create a therapeutic agent for fibromyalgia having a novel mechanism of action by the screening method using disease animal models of fibromyalgia reported in Non-Patent Document 10, the present inventors performed thorough research.

### Means for Solving the Problems

As a result, they confirmed that the administration of the 5-HT_{2C} receptor agonist produced an excellent effect of improving chronic pain in disease animal models of fibromyalgia, thereby completing the invention.
That is, the present invention relates to an agent for treating fibromyalgia, including a 5-HT_{2C} receptor agonist as an active ingredient.
The present invention also relates to a pharmaceutical composition for treating fibromyalgia, including a 5-HT_{2C} receptor agonist as an active ingredient.
The present invention also relates to the use of 5-HT_{2C} receptor agonist for producing the agent for treating fibromyalgia.
The present invention also relates to a 5-HT_{2C} receptor agonist for treating fibromyalgia.
The present invention also relates to a method for treating fibromyalgia, wherein a 5-HT_{2C} receptor agonist is administered to a patient at an effective dose.

### Effects of the Invention

According to the present invention, it is possible to provide an agent for treating fibromyalgia which is a refractory chronic disease for which a sufficiently effective therapeutic method has not been found.

### Brief Descriptions of drawings

Fig. 1 is a view showing the influence of repeated reserpine administration on the muscle tenderness threshold in male rats (A) and female rats (B).
Fig. 2 is a view showing the influence of repeated reserpine administration on the cutaneous nociception threshold in male rats (A) and female rats (B).
Fig. 3 is a view showing immobility time in forced swimming test on the first (A), third (B), fifth (C), seventh (D), and fourteenth (E) days after the repeated reserpine administration.
Fig. 4 is a view showing the effect of YM348 on the lowering of the muscle tenderness threshold induced by the repeated reserpine administration.
Fig. 5 is a view showing the effect of lorcaserin on the lowering of the muscle tenderness threshold induced by the repeated reserpine administration.
Fig. 6 is a view showing the effect of vabicaserin on the lowering of the muscle tenderness threshold induced by the repeated reserpine administration.
Fig. 7 is a view showing the antagonistic action of SB242084 on the effect of lorcaserin on the lowering of the muscle tenderness threshold induced by the repeated reserpine administration.

### Embodiments for Carrying Out the Invention

Hereinbelow, the present invention will be described in more detail.

The 5-HT_{2C} receptor agonist is a substance causing a pharmacological reaction by binding to the 5-HT_{2C} receptor. The 5-HT_{2C} receptor agonist includes a full agonist that maximally activates the receptor and a partial agonist that partially activates the receptor compared to the full agonist.
In a functional assay measuring the 5-HT_{2C} receptor activation, the 5-HT_{2C} receptor agonist preferably has an EC₅₀ of less than 10 µM, more preferably has an EC₅₀ of less than 1 µM, even more preferably has an EC₅₀ of less than 100 nM, and still more preferably has an EC₅₀ of less than 10 nM. The EC₅₀ can be measured by, for example, a functional assay or the like in a cellular system that measures the change in intracellular calcium ion concentration by using fluorescence intensity as an index.
In the inhibitory activity of the 5-HT_{2C} receptor agonist, for example, a 50% inhibitory concentration (IC₅₀) in a 5-HT_{2C} receptor binding test is preferably about less than 10 µM, more preferably about less than 1 µM, even more preferably less than 100 nM, and still more preferably less than 10 nM.

Specifically, as the 5-HT_{2C} receptor agonist, compounds disclosed in EP 0572863, EP 0863136, EP 1213017, USP 3253989, USP 3676558, USP 3652588, USP 4082844, USP 4971969, USP 5494928, USP 5646173, USP 6310208, WO 97/42183, WO 98/30546, WO 98/30548, WO 98/33504, WO 98/56768, WO 99/02159, WO 99/43647 (USP 6281243), WO 00/12475 (USP 6380238), WO 00/12502 (USP 6365598), WO 00/12510 (USP 6433175), WO 00/12475, WO 00/12481 (USP 6552062), WO 00/12482, WO 00/12502, WO 00/16761, WO 00/17170, WO 00/28993, WO 00/35922 (USP 6372745), WO 00/44737, WO 00/44753, WO 00/64899, WO 00/77001, WO 00/77002, WO 00/77010, WO 00/76984 (USP 6465467), WO 01/09111, WO 01/09122, WO 01/09123 (USP 6638936), WO 01/09126, WO 01/12602, WO 01/12603 (USP 6706750), WO 01/40183, WO 01/66548 (USP 6583134), WO 01/70207, WO 01/70223, WO 01/72752 (USP 6734301), WO 01/83487, WO 02/04456, WO 02/04457, WO 02/08178, WO 02/10169, WO 02/24700, WO 02/24701, WO 02/36596, WO 02/40456, WO 02/40457, WO 02/42304, WO 02/44152 (USP 6479534), WO 02/48124, WO 02/51844 (USP 6610685), WO 02/59124, WO 02/59127, WO 02/59129, WO 02/72584, WO 02/74746, WO 02/83863, WO 02/98350, WO 02/98400, WO 02/98860, WO 03/00663, WO 03/00666, WO 03/04501, WO 03/06466, WO 03/11281, WO 03/14118, WO 03/14125, WO 03/24976, WO 03/28733, WO 03/57161, WO 03/57213, WO 03/57673, WO 03/57674, WO 03/62205, WO 03/64423, WO 03/86306 (USP 6953787), WO 03/87086, WO 03/89409, WO 03/91250 (USP 7129237), WO 03/91251, WO 03/91257, WO 03/97636, WO 04/00829, WO 04/00830 (USP 6667303), WO 04/16256, WO 04/56324, WO 04/78718, WO 04/81010, WO 04/087156, WO 04/87662, WO 04/87692, WO 04/89897, WO 04/096196, WO 04/96201, WO 04/112769, US 2004192754, WO 05/00849, WO 05/03096, EP 1500391, WO 05/16902, WO 05/19180, US 2005080074, WO 05/35533, WO 05/40146, WO 05/41856, WO 05/42490, WO 05/42491, WO 05/44812, WO 06/04931, WO 06/28961, WO 06/65600, WO 06/65706, WO 06/86464, WO 06/103511, WO 06/116148, WO 06/116149, WO 06/116150, WO 06/116165, WO 06/116170, WO 06/116171, WO 07/25144, WO 07/84622, WO 07/123941, WO 08/09125, WO 08/52086, WO 08/52087, WO 08/52088, USP 3652543, WO 08/117169, WO 09/79765, and the like can be used.

Among these, particularly the following compounds (1) to (89) and the like are preferably used. The chemical structures are shown in Tables 1 to 8.
(1) YM348
(2) Lorcaserin hydrochloride (APD-356)
(3) Vabicaserin hydrochloride (SCA-136)
(4) Compound represented by the structural formula in Table 1 disclosed in WO 07/84622
(5) Compound represented by the structural formula in Table 1 disclosed in WO 06/04931
(6) Compound represented by the structural formula in Table 1 disclosed in WO 06/86464
(7) PF-3246799
(8) WAY-211229
(9) WAY-261240
(10) WAY-163909
(11) Compound represented by the structural formula of Table 2 disclosed in WO 05/42491
(12) PNU-22394A
(13) Ro60-0175
(14) ORG-12962
(15) ALX-2226
(16) Ro60-0332
(17) VER-2692
(18) VER-3993
(19) VER-5593
(20) VER-5384
(21) VER-3323
(22) PNU-57378E
(23) WAY-629
(24) WAY-161503
(25) Compound represented by the structural formula of Table 3 disclosed in WO 06/65706
(26) Compound represented by the structural formula of Table 3 disclosed in WO 06/65600
(27) Compound represented by the structural formula of Table 3 disclosed in WO 06/28961
(28) Compound represented by the structural formula of Table 3 disclosed in WO 09/76765
(29) Compound represented by the structural formula of Table 3 disclosed in WO 08/09125
(30) Compound represented by the structural formula of Table 3 disclosed in WO 06/116150
(31) Compound represented by the structural formula of Table 3 disclosed in WO 08/52086
(32) m-Chlorophenylpiperazine (m-CPP)
(33) PAL-287
(34) MK-212
(35) ALX-2218
(36) VER-6925
(37) VER-7397
(38) VER-7499
(39) VER-7443
(40) PNU-243922
(41) Org-37684
(42) Org-36262 (Ro60-0527)
(43) Ro60-0017 (Org-35013)
(44) VER-3881
(45) Ro0721256
(46) PNU-181731A
(47) IL-639
(48) IK-264
(49) Ro60-0759
(50) Ro60-0869
(51) WAY-470
(52) WAY-162545
(53) IX-065
(54) A-37215
(55) Compound represented by the structural formula of Table 6 disclosed in WO 00/12510
(56) Compound represented by the structural formula of Table 6 disclosed in WO 02/51844
(57) Compound represented by the structural formula of Table 6 disclosed in WO 01/66548
(58) Compound represented by the structural formula of Table 6 disclosed in WO 00/12482
(59) Compound represented by the structural formula of Table 6 disclosed in WO 03/24976
(60) Compound represented by the structural formula of Table 6 disclosed in WO 03/57213
(61) Compound represented by the structural formula of Table 6 disclosed in WO 03/57674
(62) Compound represented by the structural formula of Table 6 disclosed in WO 02/98860
(63) Compound represented by the structural formula of Table 6 disclosed in WO 02/44152
(64) Compound represented by the structural formula of Table 6 disclosed in WO 02/44737
(65) Compound represented by the structural formula of Table 7 disclosed in WO 02/40456
(66) Compound represented by the structural formula of Table 7 disclosed in WO 02/08178
(67) Compound represented by the structural formula of Table 7 disclosed in WO 03/00666
(68) Compound represented by the structural formula of Table 7 disclosed in WO 01/09123
(69) Compound represented by the structural formula of Table 7 disclosed in WO 01/09122
(70) Compound represented by the structural formula of Table 7 disclosed in WO 01/09126
(71) Compound represented by the structural formula of Table 7 disclosed in EP 0572863
(72) Compound represented by the structural formula of Table 7 disclosed in WO 02/72584
(73) Compound represented by the structural formula of Table 7 disclosed in WO 02/59127
(74) Compound represented by the structural formula of Table 7 disclosed in WO 03/14118
(76) Compound represented by the structural formula of Table 8 disclosed in WO 00/64899
(77) Compound represented by the structural formula of Table 8 disclosed in WO 03/06466
(78) Compound represented by the structural formula of Table 8 disclosed in WO 02/74746
(79) Compound represented by the structural formula of Table 8 disclosed in WO 02/42304
(80) Compound represented by the structural formula of Table 8 disclosed in WO 97/42183
(81) Compound represented by the structural formula of Table 8 disclosed in WO 02/48124
(82) Compound represented by the structural formula of Table 8 disclosed in WO 05/16902
(83) Compound represented by the structural formula of Table 8 disclosed in WO 04/99150
(84) Compound represented by the structural formula of Table 8 disclosed in USP 3652543
(85) Nordexfenfluramine
(86) Oxaflozane
(87) AR-10A
(88) BVT-933
(89) VR-1065

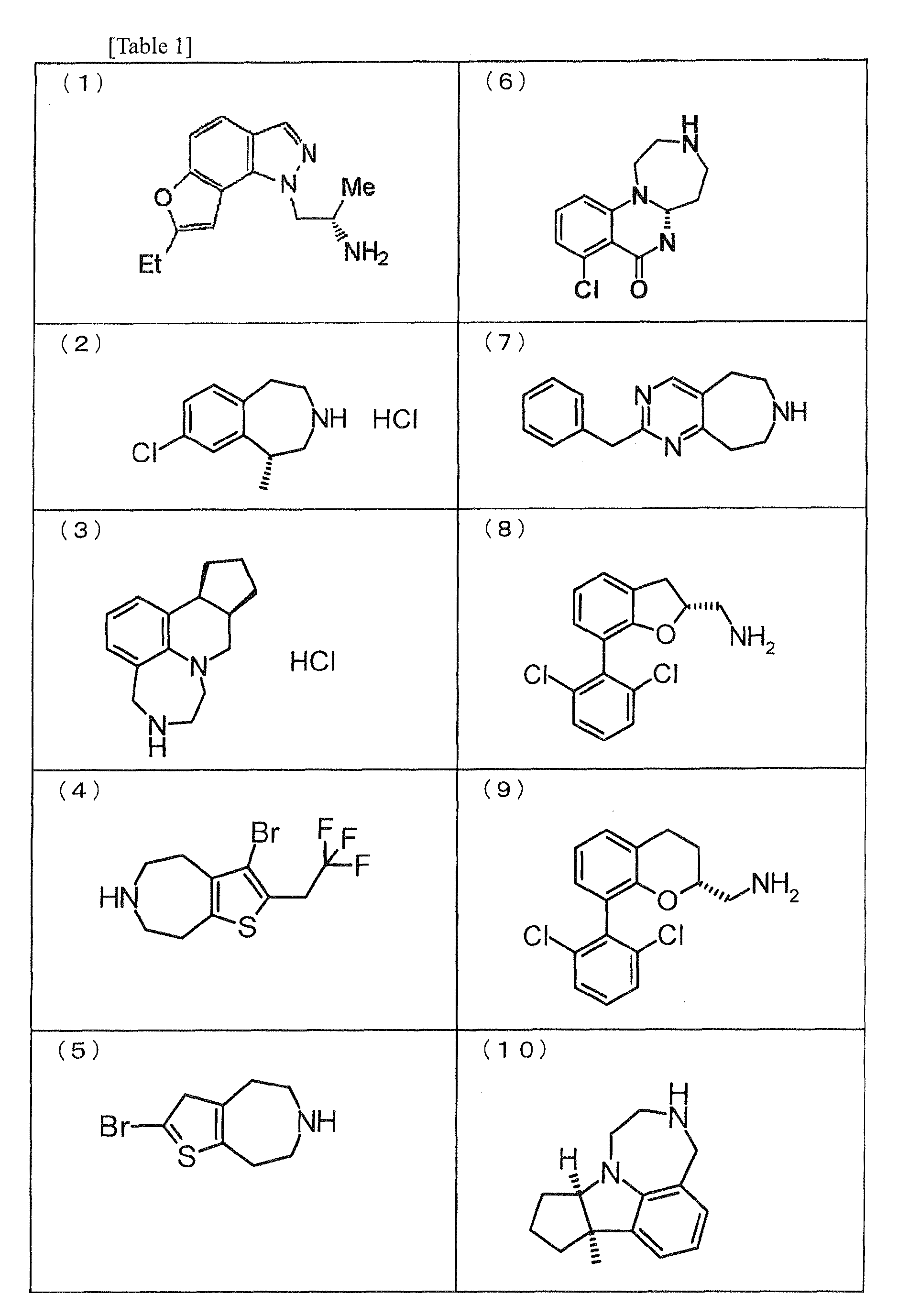

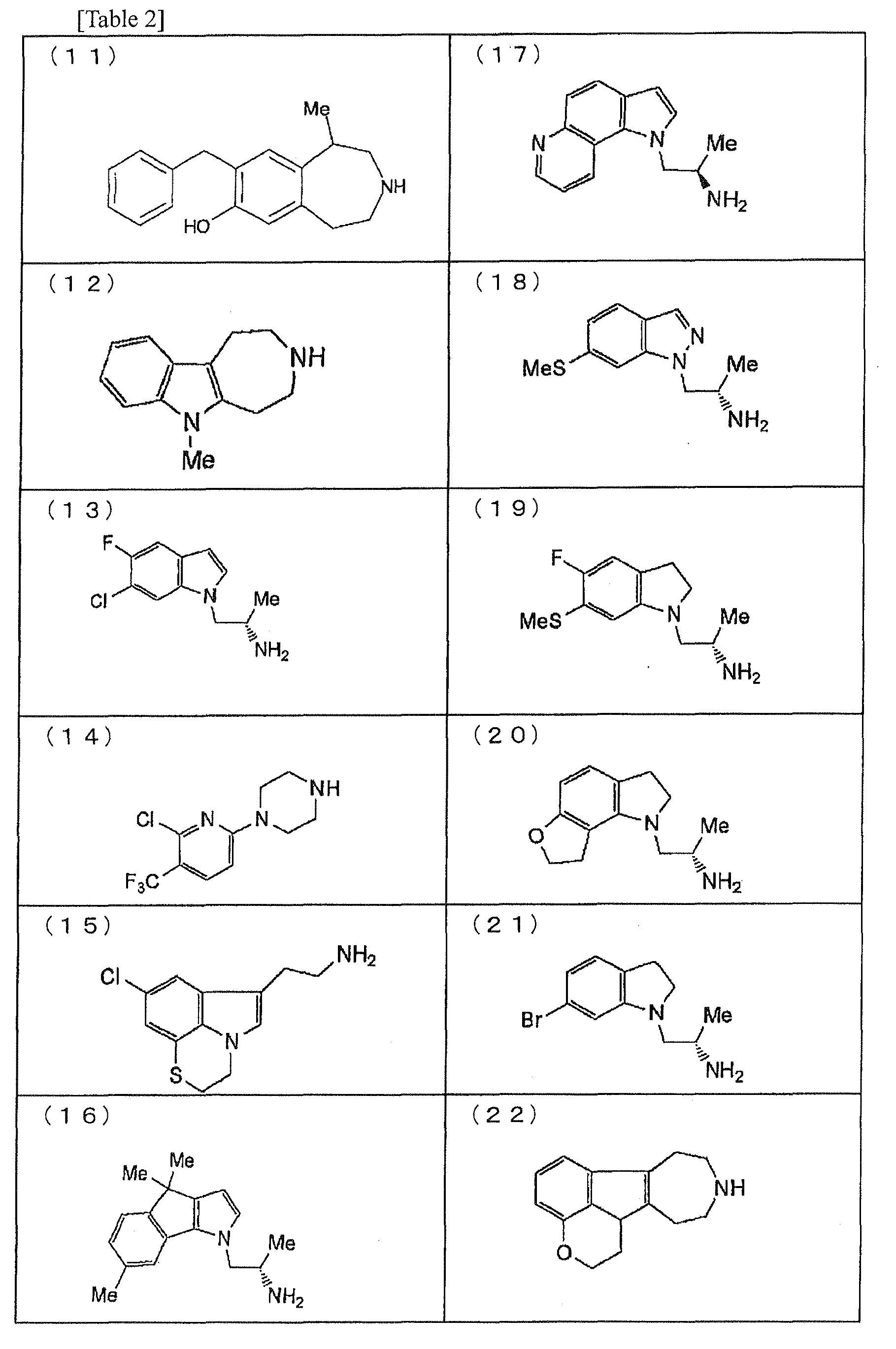

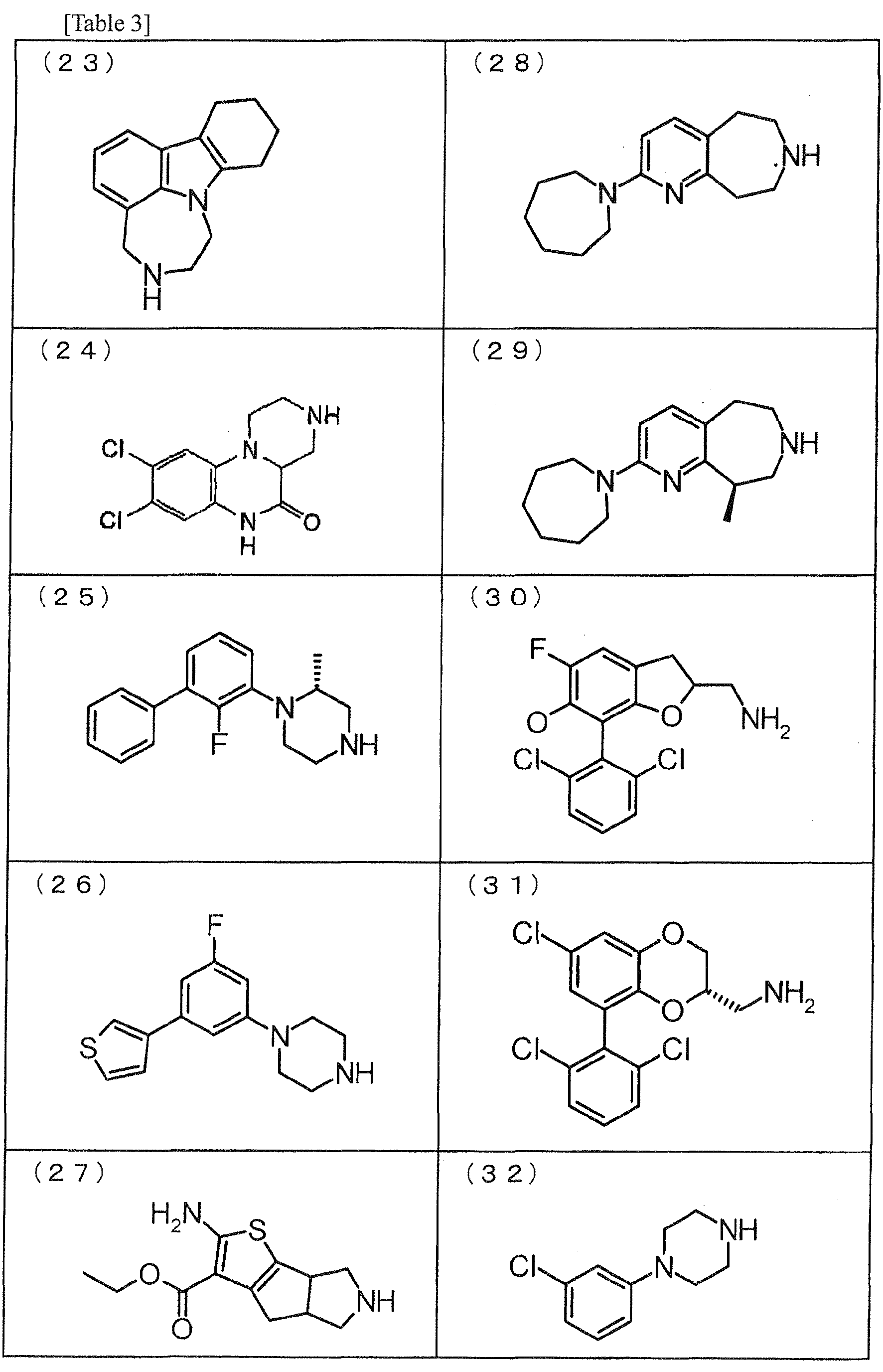

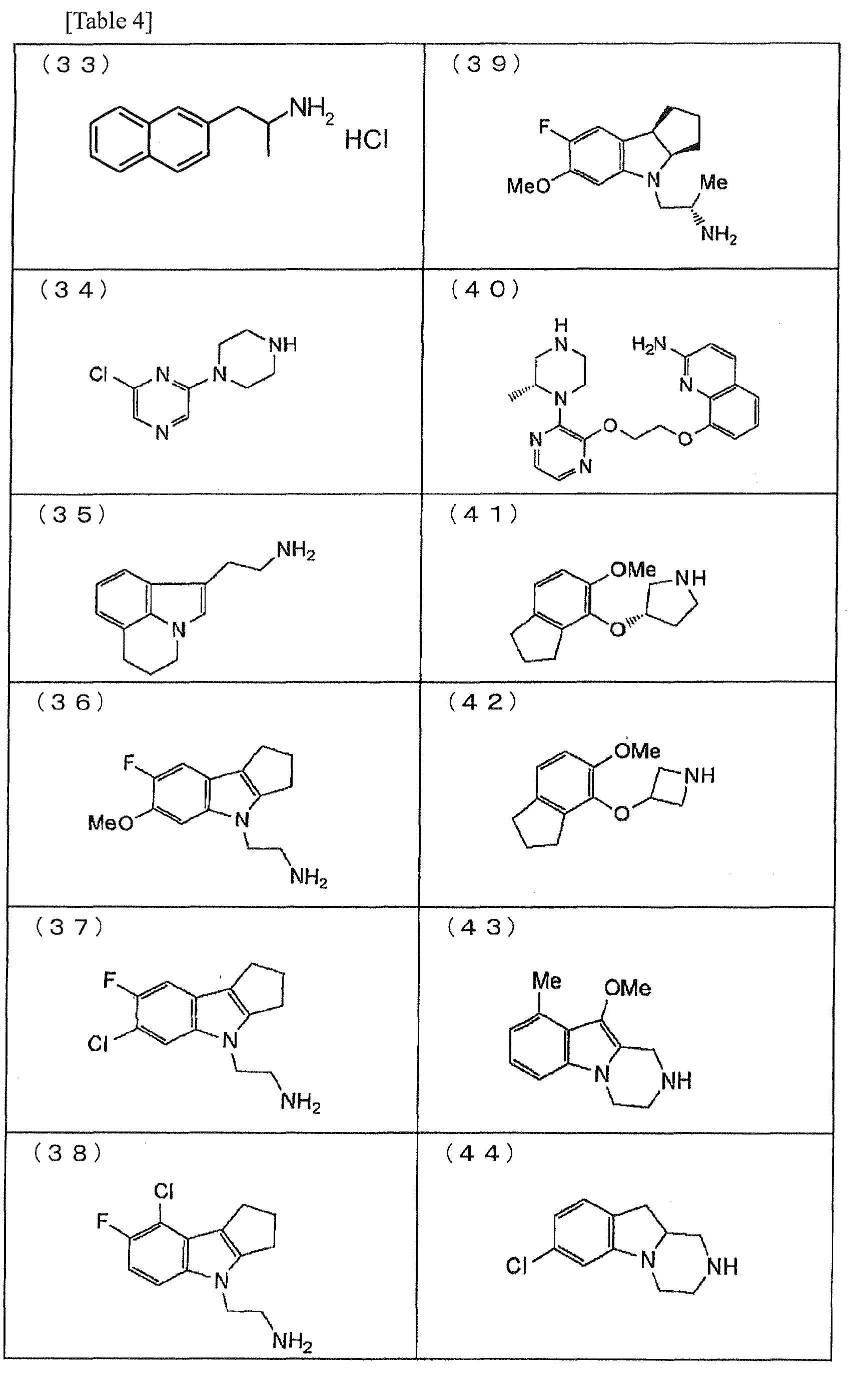

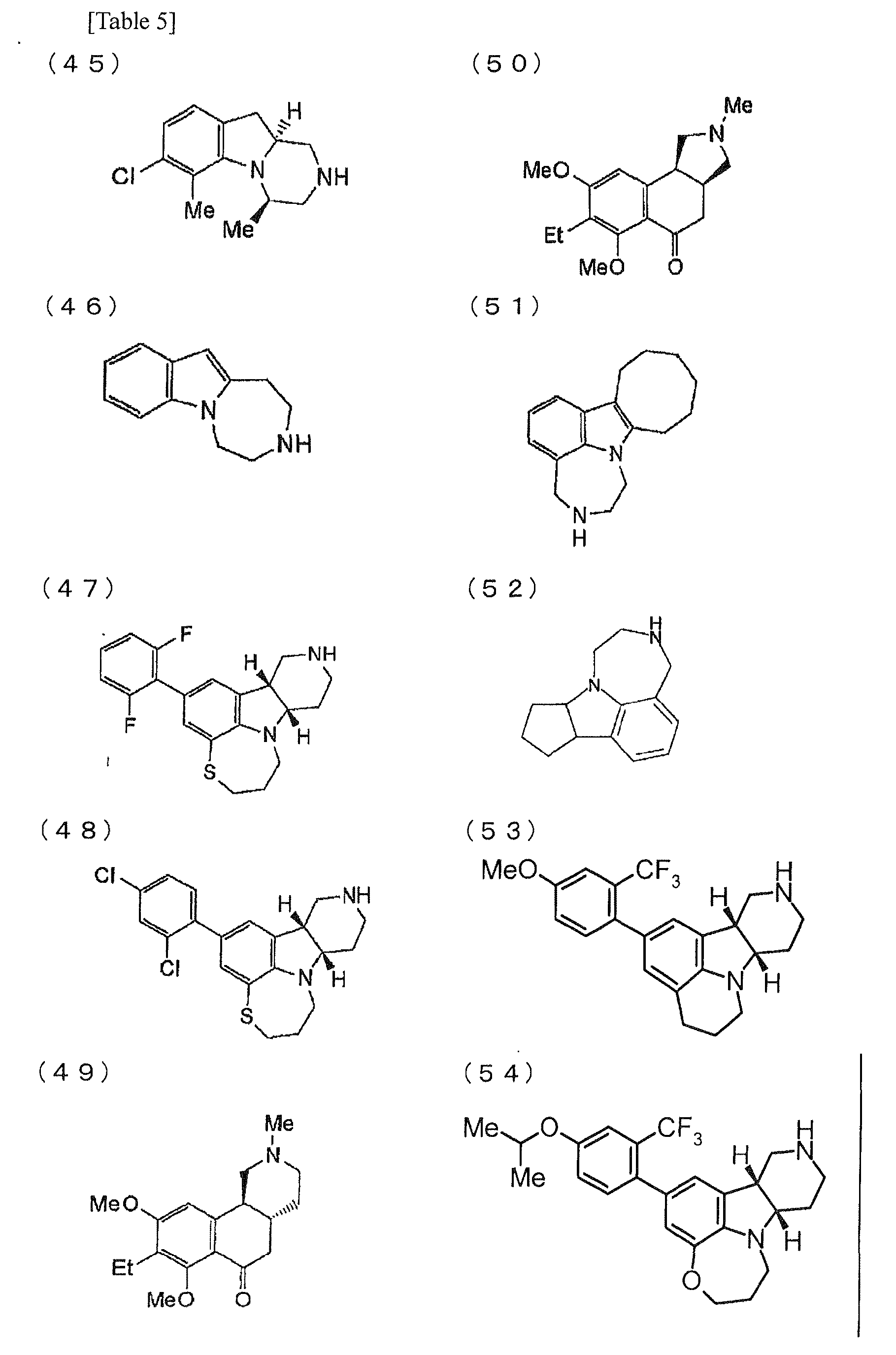

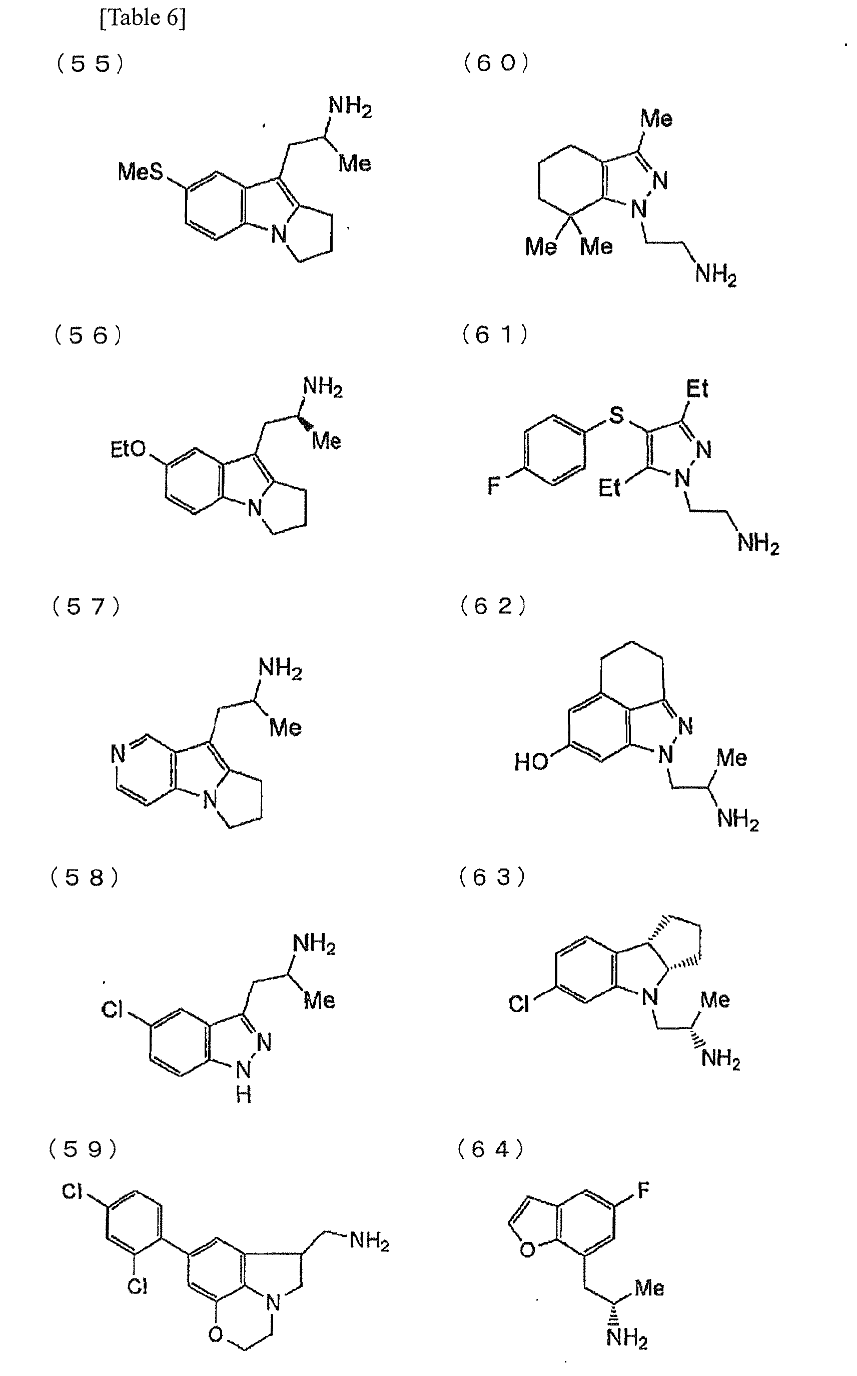

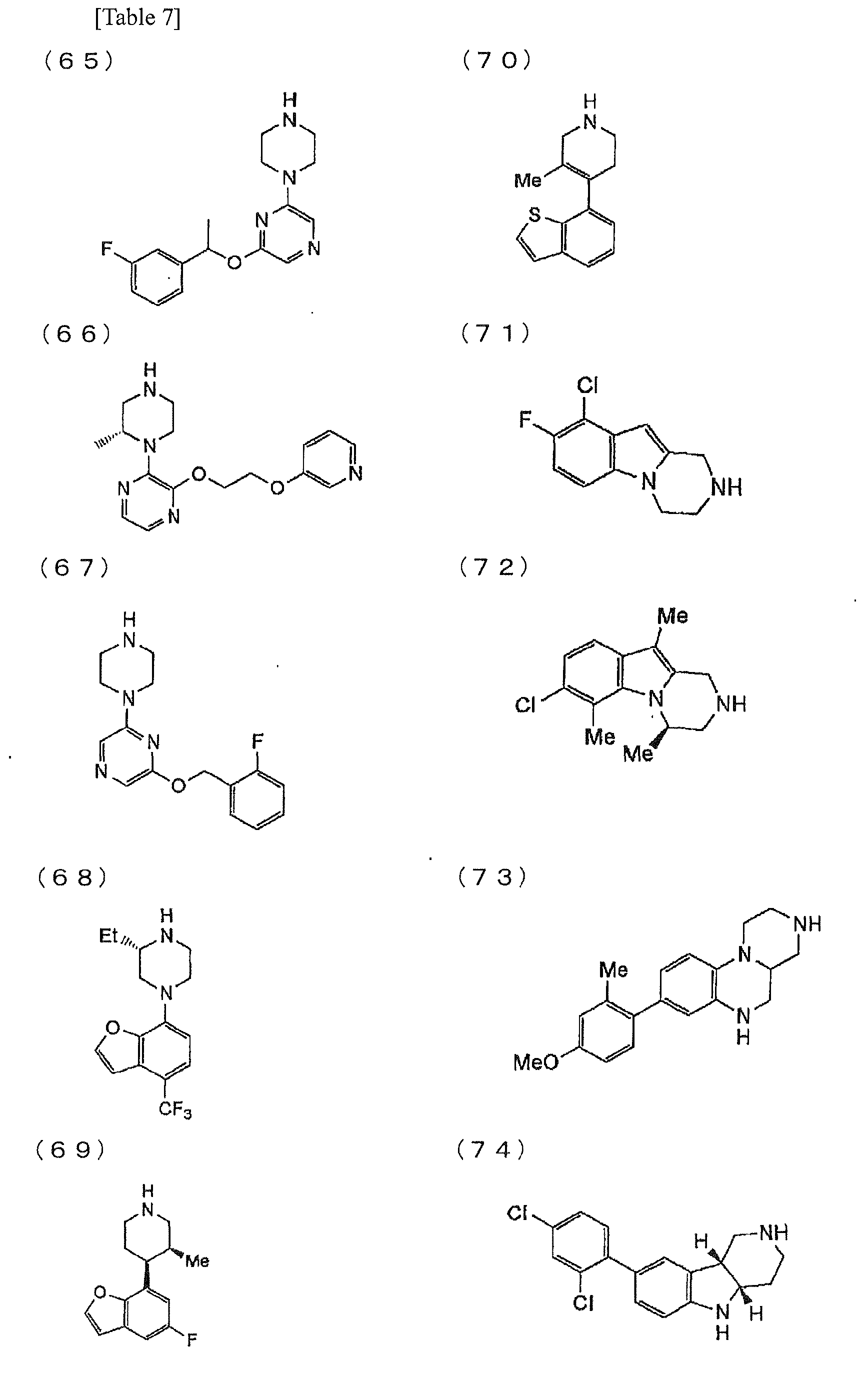

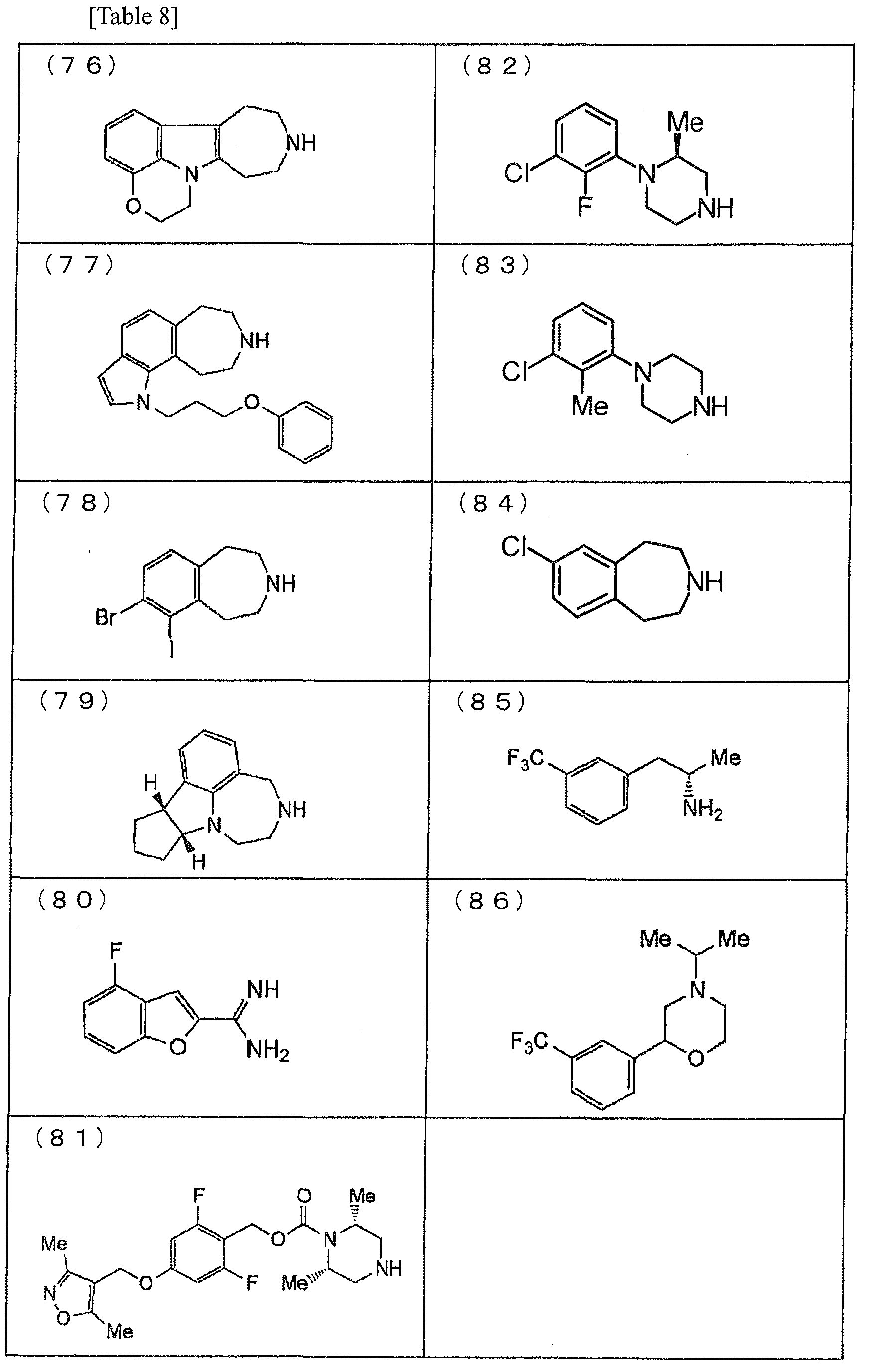

Among the compounds (1) to (89), compounds (1) to (74) and (76) to (84) are preferable, compounds (1) to (54) are more preferable, and compounds (1) to (31) are most preferable.

The 5-HT_{2C} receptor agonist preferably and appropriately used for the present invention also includes the compounds included in Patent Document 1 (preferably the compounds exemplified in Patent Document 1). These include a compound represented by Formula (I) or a pharmaceutically acceptable salt thereof, 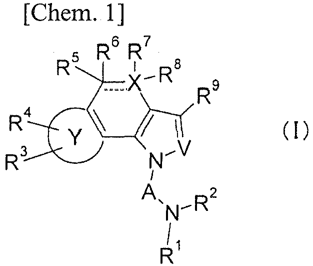 (symbols in the formula mean the following:
ring Y represents an unsaturated 5-membered hetero ring which may have 1 to 3 of one or two or more kinds of hetero atoms selected from N, O and S atoms, or represents an unsaturated 6-membered hetero ring having 1 to 2 N atoms;
X represents a bond or C;
V represents N or CH;
A represents linear or branched C₁₋₆ alkylene which may be substituted with halogen or C₃₋₈ cycloalkyl;
R¹ and R² may be the same as or different from each other and represent H or C₁₋₆ alkyl, alternatively, R¹ and R² or R¹ and A may form a 3- to 8-membered nitrogen-containing saturated hetero ring together with an N atom to which R¹ and R² or R¹ and A bind;
R³ and R⁴ may be the same as or different from each other and represent H, C₁₋₆ alkyl, OH, C₁₋₆ alkyl-O-, amino, C₁₋₆ alkyl-NH-, (C₁₋₆ alkyl)₂-N-, C₁₋₆ alkyl-CO-NH-, or halogen;
R⁵ to R⁹ may be the same as or different from each other and represent H, C₁₋₆ alkyl, OH, or C₁₋₆ alkyl-O-; and
the portion of a dotted line represents an arbitrary double bond;
provided that, when the portion of a dotted line is a double bond, R⁶ and R⁸ do not exist, and when X is a bond, the portion of a dotted line is a single bond, and R⁷ and R⁸ do not exist).

Among compounds represented by Formula (I), the compounds included in Patent Document 2 (preferably, the compounds exemplified in Patent Document 2) are more preferable. These include a compound represented by Formula (I') or a pharmaceutically acceptable salt thereof. 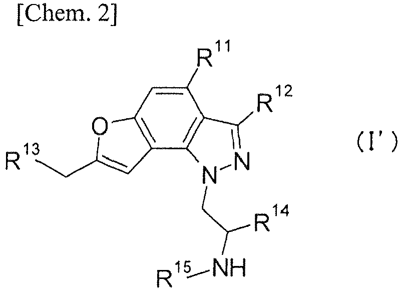 (symbols in the formula mean the following:
R¹¹, R¹², R¹⁴, and R¹⁵ may be the same as or different from each other and represent H or C₁₋₆ alkyl; and
R¹³ represents C₁₋₅ alkyl)
Examples of preferable compounds represented by Formula (I') include, but are limited to, the following compounds or pharmaceutically acceptable salts thereof,
(S)-2-(7-ethyl-1H-furo[2,3-g]indazol-1-yl)-1-methylethylamine;
(S)-2-(7-ethyl-3-methyl-1H-furo[2,3-g]indazol-1-yl)-1-methylethylamine;
(S)-2-(3,7-diethyl-1H-furo[2,3-g]indazol-1-yl)-1-methylethylamine;
(S)-1-methyl-2-(7-propyl-1H-furo[2,3-g]indazol-1-yl)ethylamine;
(S)-N-ethyl-2-(7-ethyl-1H-furo[2,3-g]indazol-1-yl)-methylethylamine;
(S)-2-(4,7-diethyl-1H-furo[2,3-g]indazol-1-yl)-1-methylethylamine;
(S)-2-(7-butyl-1H-furo[2,3-g]indazol-1-yl)-1-methylethylamine; or
(S)-2-(7-isopentyl-1H-furo[2,3-g]indazol-1-yl)-1-methylethylamine.

Among the 5-HT_{2C} receptor agonists represented by Formula (I'), (S)-2-(7-ethyl-1H-furo[2,3-g]indazol-1-yl)-1-methylethylamine (YM348) or a pharmaceutically acceptable salt thereof is particularly preferable.

The 5-HT_{2C} receptor agonist preferably and appropriately used for the present invention also includes the compounds included in Patent Document 3 (preferably, the compounds exemplified in Patent Document 3). These include a compound represented by Formula (II) or a pharmaceutically acceptable salt thereof, 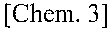 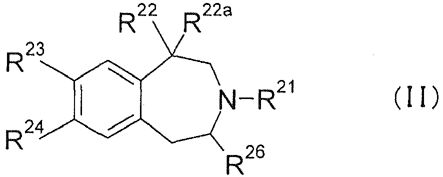 (symbols in the formula mean the following:
R²¹ represents H or C₁₋₈ alkyl;
R²² represents C₁₋₈ alkyl, -CH₂-O-C₁₋₈ alkyl, -C(=O)-O-C₁₋₈ alkyl, -C(=O)-NH-C₁₋₈ alkyl, OH, or CH₂OH;
R^{22a} represents H, alternatively, R²² and R^{2a} form -CH₂-CH₂- together;
R²³ represents halogen, perhalo-C₁₋₈ alkyl, CN, -SR²⁵, -NHR²⁵, -N(R²⁵)₂, aryl, or heteroaryl, wherein the aryl may be optionally substituted with one or two substituents selected from C₁₋₈ alkyl, halogen, perhalo-C₁₋₈ alkyl, and C₁₋₈ alkyl-O-, and the heteroaryl may be optionally substituted with one or two substituents selected from halogen and C₁₋₈ alkyl;
R²⁴ represents H, halogen, perhalo-C₁₋₈ alkyl, CN, -SR²⁵, -NHR²⁵, -N(R²⁵)₂, aryl, or heteroaryl, whrein the aryl may be optionally substituted with one or two substituents selected from C₁₋₈ alkyl, halogen, perhalo-C₁₋₈ alkyl, and C₁₋₈ alkyl-O-, and the heteroaryl may be optionally substituted with one or two substituents selected from halogen and C₁₋₈ alkyl;
alternatively, R²³ and R²⁴ may form a 5- or 6-membered hetero ring having one O atom together with an atom to which R²³ and R²⁴ bind;
R²⁵ independently represents C₁₋₈ alkyl, C₁₋₈ alkenyl, aryl, heteroaryl, aryl-C₁₋₈ alkyl-, heteroaryl-C₁₋₈ alkyl- or perhalo-C₁₋₈ alkyl, or allyl; and
R²⁶ represents H or C₁₋₈ alkyl;
provided that, when R²⁶ is C₁₋₈ alkyl, R²⁴ represents a group other than H).

Examples of preferable compounds represented by Formula (II) include, but are not limited to, the following compounds or pharmaceutically acceptable salts thereof,
8-chloro-1-methyl-2,3,4,5-tetrahydro-1H-3-benzazepine;
8-bromo-1-methyl-2,3,4,5-tetrahydro-1H-3-benzazepine;
8-iodo-1-methyl-2,3,4,5-tetrahydro-1H-3-benzazepine;
8-trifluoromethyl-1-methyl-2,3,4,5-tetrahydro-1H-3-benzazepine;
8-trifluoromethyl-1-ethyl-2,3,4,5-tetrahydro-1H-3-benzazepine;
8-chloro-1-ethyl-2,3,4,5-tetrahydro-1H-3-benzazepine;
8-bromo-1-ethyl-2,3,4,5-tetrahydro-1H-3-benzazepine;
8-iodo-1-ethyl-2,3,4,5-tetrahydro-1H-3-benzazepine;
7,8-dichloro-1-methyl-2,3,4,5-tetrahydro-1H-3-benzazepine;
7,8-dichloro-1-ethyl-2,3,4,5-tetrahydro-1H-3-benzazepine;
8-chloro-7-fluoro-1-methyl-2,3,4,5-tetrahydro-1H-3-benzazepine; or
8-chloro-7-fluoro-1-ethyl-2,3,4,5-tetrahydro-1H-3-benzazepine.

Among the 5-HT_{2C} receptor agonists represented by Formula (II), (1R)-8-chloro-1-methyl-2,3,4,5-tetrahydro-1H-3-benzazepine (generic name: lorcaserin) or a pharmaceutically acceptable salt thereof is particularly preferable.

The 5-HT_{2C} receptor agonist preferably and appropriately used for the present invention also includes the compounds included in Patent Document 4 (preferably, the compounds exemplified in Patent Document 4). These include a compound represented by Formula (III) or a pharmaceutically acceptable salt thereof, 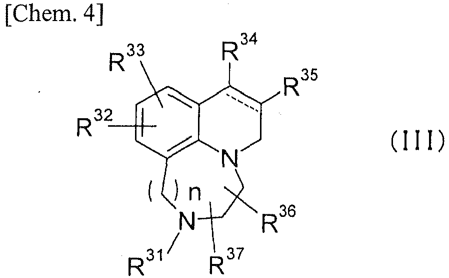 (in the formula,
R³¹ represents H, C₁₋₆ alkyl, C₁₋₅ alkyl-C(=O)-, or phenyl-C₁₋₆ alkyl-O-C(=O)-;
each of R³² and R³³ independently represents H, OH, C₁₋₆ alkyl, C₁₋₆ alkyl-O-, halogen, NH₂-C(=O)-, C₁₋₅ alkyl-O-C(=O)-, perfluoro-C₁₋₆ alkyl, cyano, C₁₋₆ alkyl-S(O)₂-NH-, C₁₋₆ alkyl-S(O)₂-, C₁₋₅ alkyl-C(=O)-NH-, amino, C₁₋₆ alkyl-NH-, (C₁₋₆ alkyl)₂-N-, perfluoro-C₁₋₆ alkyl-O-, C₁₋₅ alkyl-C(=O)-O-, C₁₋₅ alkyl-C(=O)-, phenyl-C(=O)-, phenyl, phenyl-C₁₋₆ alkyl-, heteroaryl, or heteroaryl-C₁₋₆ alkyl-, wherein , a phenyl or heteroaryl portion of all substituents having the phenyl or heteroaryl portion may be substituted with 1 to 3 substituents independently selected from halogen, C₁₋₆ alkyl, or C₁₋₆ alkyl-O-;
each of R³⁴ and R³⁵ independently represents H or C₁₋₆ alkyl, alternatively, R³⁴ and R³⁵ may form a cyclic portion selected from monocyclic C₄₋₈ alkane, monocyclic C₄₋₈ alkene, bridged bicyclic C₅₋₁₀ alkane, bridged bicyclic C₅₋₁₀ alkene, pyran, and thiopyran (wherein, a S atom may be oxidized to SO or SO₂) together with carbon to which R³⁴ and R³⁵ bind, wherein, the cyclic portion formed by R³⁴ and R³⁵ may be substituted with 1 to 3 substituents selected from halogen, C₁₋₆ alkyl and C₁₋₆ alkyl-O-;
each of R³⁶ and R³⁷ independently represents H or C₁₋₆ alkyl; and
n represents 1 or 2;
provided that, the portion of a dotted line represents an arbitrary double bond;
wherein, heteroaryl means a 5- to 7-membered monocyclic aromatic ring group having 1 or 2 hetero atoms selected from N, O, and S).

Examples of the preferable compound represented by Formula (III) include, but are not limited to, the following compounds or pharmaceutically acceptable salts thereof.
4,5,6,7,9,9a,10,11,12,12a-decahydrocyclopenta[c][1,4]diazepino[6,7,1-ij]quinoline;
2-bromo-4,5,6,7,9,9a, 10,11,12,12a-decahydrocyclopenta[c] [1,4]diazepino[6,7,1-ij]quinoline;
2-chloro-4,5,6,7,9,9a,10,11,12,12a-decahydrocyclopenta[c] [1,4]diazepino[6,7,1-ij]quinoline;
2-phenyl-4,5,6,7,9,9a, 10,11,12,12a-decahydrocyclopenta[c] [1,4]diazepino[6,7,1-ij]quinoline;
2-methoxy-4,5,6,7,9,9a,10,11,12,12a-decahydrocyclopenta[c][1,4]diazepino[6,7,1-ij]quinoline;
1-fluoro-4,5,6,7,9,9a,10,11,12,12a-decahydrocyclopenta[c][1,4]diazepino[6,7,1 - ij]quinoline;
1-(trifluoromethyl)-4,5,6,7,9,9a,10,11,12,12a-decahydrocyclopenta[c][1,4]diazepino[6,7,1-ij]quinoline; or
1-fluoro-2-methoxy-4,5,6,7,9,9a,10,11,12,12a-decahydrocyclopenta[c][1,4]diazepino[6,7,1-ij]quinoline

Among the 5-HT_{2C} receptor agonists represented by Formula (III), (-)-(9aR, 12aS)-4,5,6,7,9,9a, 10,11,12,12a-decahydrocyclopenta[c][1,4]diazepino[6,7,1-ij]quinoline (generic name: vabicaserin) or a pharmaceutically acceptable salt thereof is particularly preferable.

The 5-HT_{2C} receptor agonist particularly preferably and appropriately used for the present invention is
(S)-2-(7-ethyl-1H-furo[2,3-g]indazol-1-yl)-1-methylethylamine (YM348);
(1R)-8-chloro-1-methyl-2,3,4,5-tetrahydro-1H-3-benzazepine (generic name: lorcaserin);
(-)-(9aR, 12aS)-4,5,6,7,9,9a,10,11,12,12a-decahydrocyclopenta[c][1,4]diazepino[6,7,1-ij]quinoline (generic name: vabicaserin);
or a pharmaceutically acceptable salt thereof.

The various terms in the definitions of the above general formulae mean the following unless otherwise sepcified.
Alkyl is a linear or branched aliphatic saturated hydrocarbon group, and examples thereof include methyl, ethyl, n-propyl, isopropyl, n-butyl, isobutyl, sec-butyl, tert-butyl, n-pentyl, n-hexyl, and the like.
Alkenyl is a linear or branched aliphatic saturated hydrocarbon group, and examples thereof include vinyl, propenyl, butenyl, pentenyl, 1-methylvinyl, 1-methyl-2-propenyl, 1,3-butadienyl, 1,3-pentadienyl, and the like.
Cycloalkyl is an aliphatic saturated hydrocarbon ring group which may have a bridge, and examples thereof include cyclopropyl, cyclobutyl, cyclopentyl, cyclohexyl, cycloheptyl, cyclooctyl, adamantyl, and the like.
Cycloalkane is an aliphatic saturated C₃₋₁₀ hydrocarbon ring which may have a bridge and includes monocyclic C₄₋₈ alkane or bridged bicyclic C₅₋₁₀ alkane.
Cycloalkene is an aliphatic unsaturated C₃₋₁₀ hydrocarbon ring which may have a bridge and includes monocyclic C₄₋₈ alkene or bridged bicyclic C₅₋₁₀ alkene.
Aryl is a mono- to tricyclic aromatic C₆₋₁₄ hydrocarbon ring group, and examples thereof include phenyl and naphthyl. As another embodiment, aryl is phenyl.
A hetero ring is a 3- to 15-membered mono- to tricyclic hetero ring group having 1 to 4 hetero atoms selected from N, O, and S and includes a saturated ring, an aromatic ring, and a partially hydrogenated ring group thereof. S or N which is a ring atom may form an oxide or a dioxide by being oxidized. Heteroaryl is a monocyclic aromatic ring group or a bi- or tricyclic unsaturated ring group having at least one aromatic ring as a constituent element, among the above hetero rings. Accordingly, a hetero ring is specifically monocylcic heteroaryl such as pyridyl, pyrrolyl, pyrazinyl, pyrimidinyl, pyridazinyl, imidazolyl, triazolyl, triazinyl, tetrazolyl, thiazolyl, pyrazolyl, isothiazolyl, oxazolyl, isoxazolyl, thiadiazolyl, oxadiazolyl, thienyl, or furyl; bicyclic heteroaryl such as indolyl, isoindolyl, benzimidazolyl, indazolyl, quinolyl, isoquinolyl, quinazolinyl, quinoxalinyl, phthalazinyl, benzothiazolyl, benzisothiazolyl, benzothiadiazolyl, benzoxazolyl, benzisoxazolyl, benzofuranyl, or benzothienyl; tricyclic heteroaryl such as carbazolyl, dibenzo[b,d]furanyl, or dibenzo[b,d]thienyl; a non-aromatic monocyclic hetero ring such as azetidinyl, pyrrolidinyl, piperidinyl, piperazinyl, azepanyl, diazepanyl, morpholinyl, thiomorpholinyl, tetrahydropyridinyl, oxetanyl, tetrahydrofuranyl, tetrahydropyranyl, dioxolanyl, dioxanyl, or tetrahydrothiopyranyl; a non-aromatic bicyclic hetero ring such as indolinyl, tetrahydroquinolyl, tetrahydroisoquinolyl, dihydrobenzimidazolyl, tetrahydrobenzimidazolyl, tetrahydroquinoxalinyl, dihydroquinoxalinyl, dihydrobenzoxazolyl, dihydrobenzoxazinyl, dihydrobenzofuryl, chromanyl, chromenyl, methylenedioxyphenyl, or ethylenedioxyphenyl; a bridged hetero ring such as quinuclidinyl, or the like.
Specific examples of the unsaturated 5-membered hetero ring which may have 1 to 3 of 1 or 2 or more kinds of hetero atoms selected from N, O and S atoms include pentene, pentadiene, thiophene, furan, pyrrole, imidazole, pyrazole, thiazole, oxazole, isothiazole, isoxazole, triazole, thiadiazole, oxadiazole, and the like.
Specific examples of the unsaturated 6-membered hetero ring having 1 to 2 N atoms include pyridine, pyridazine, pyrimidine, pyrazine, and the like.
Examples of the 3- to 8-membered nitrogen-containing saturated hetero ring include aziridine, azetidine, pyrrolidine, piperidine, hexahydroazepine, octahydroazocine, and the like.
Halogen or halo means a Cl, Br, F, or I atom.
Perhalo-C₁₋₆ alkyl means alkyl having 1 to 6 carbon atoms in which 3 or more of the hydrogen atoms are substituted with halogen.
A pharmaceutically acceptable salt means a pharmaceutically acceptable acid or base addition salt with an organic or inorganic acid or base, and examples thereof include a mineral acid salt with hydrochloric acid, sulfuric acid, phosphoric acid, hydrobromic acid, or the like; a salt with an organic acid such as acetic acid, oxalic acid, succinic acid, citric acid, maleic acid, malic acid, fumaric acid, tartaric acid, or methanesulfonic acid; and a salt with an acidic amino acid such as glutamic acid or asparaginic acid.

These 5-HT_{2C} receptor agonists which are existing compounds can be easily obtained by the method disclosed in the above documents or obtained based on the method.

The dose of the active ingredient of the present invention is appropriately determined for each medicinal agent on a case by case basis in consideration of the route of administration, the symptoms of a disease, the age, sex, and the like of an administration target, within a range of a daily dose of from 0.01 mg to 1000 mg. In addition, from the results of experiments performed on disease animal models shown in Example 1 described later, a clinically effective dose for a patient is converted into the following preferable range. In a case of oral administration, YM348 is preferably administered at about 0.1 mg to 100 mg/day to an adult in a single dose or in several separate doses per day. In a case of oral administration, lorcaserin is preferably administered at about 0.1 mg to 1000 mg/day to an adult in a single dose or in several divided doses per day. In a case of oral administration, vabicaserin is preferably administered at about 1 mg to 1000 mg/day to an adult in a single dose or in several divided doses per day.

The medicinal agent of the present invention can be prepared as an oral solid preparation, an oral liquid preparation, or an injection according to common methods by using an organic or inorganic carrier, an excipient, and other additives appropriate for oral or parenteral administration. Among these, an oral solid preparation that can easily be taken by patient him or herself and can be conveniently stored and carried is preferable. Specific examples of the oral solid administration include a tablet, powder, granules, fine granules, a capsule, a pill, and the like.
In the solid preparation, an active substance is mixed with at least an inactive diluent, for example, lactose, mannitol, glucose, microcrystalline cellulose, starch, polyvinyl pyrrolidone, or magnesium metasilicate aluminate. The composition may contain additives other than the inactive diluents, for example, a binder such as hydroxypropyl cellulose or hydroxypropyl methylcellulose, a lubricant such as magnesium stearate, calcium stearate, polyethylene glycol, starch, or talc, a disintegrator such as calcium cellulose glycolate, a stabilizer such as lactose, a dissolution adjuvant such as glutamic acid or asparaginic acid, Tween 80, a plasticizer such as triacetin, and a colorant such as titanium oxide or iron sesquioxide, according to common methods. The tablet or pill may be optionally coated with a sugar film or a film of a gastric-soluble or an enteric-soluble substance such as sucrose, gelatin, agar, pectin, hydroxypropyl cellulose, or hydroxypropyl methylcellulose phthalate.

The therapeutic effect of the 5-HT_{2C} receptor agonist on fibromyalgia can be confirmed by the effect of improving the muscle tenderness threshold that is an index of chronic myalgia for the disease animal model of Non-Patent Document 10, as shown in examples. In addition, the effect can be evaluated by measuring one or two or more items among the muscle tenderness threshold as in index of chronic myalgia, the cutaneous nociception threshold as an index of tactile allodynia, cold sensitivity as an index of cold allodynia, and thermosensitivity as an index of thermal hyperalgesia. The effect can also be evaluated by plantar stimulation caused by a Randall Selitto instrument, the lowering of a reaction threshold with respect to mechanical stimulation such as pinch stimulation, the lowering of a reaction threshold with respect to thermal stimulation, the lowering of a reaction threshold with respect to cold stimulation, the lowering of a reaction threshold with respect to electric stimulation, the increase in pain behavior caused by chemical stimulation, the increase in spontaneous pain behavior caused when a stimulus is not input, such as the fluctuation of spontaneous motor activity or limb-lifting behavior, and the like.
The muscle tenderness threshold refers to the magnitude of a minimum pressure stimulus that causes an animal to show a withdrawal response when a slowly increasing pressure stimulus is applied to the muscle. In order to measure the threshold, it is possible to select and use various known methods according to the type of animals. For example, in a case of a rat, it is possible to use the method of Schafers et al, that is, a method of applying a slowly increasing pressure stimulus to the gastrocnemius of a right rear foot and measuring the magnitude of the minimum pressure stimulus at which the rat shows the withdrawal response as the muscle tenderness threshold, as shown in examples and the like described later.
The threshold of cutaneous nociception refers to the magnitude of a minimum tactile stimulus that causes an animal to show a withdrawal reflex when a gradually increasing tactile stimulus is applied to the skin. In order to measure the threshold, it is possible to select and use various known methods according to the type of animals. For example, in a case of a rat, it is possible to use a von Frey hair method in which von Frey filaments differing in thickness are applied to the plantar surface of a right rear foot of a rat, whereby a filament having the smallest thickness that causes the rat to show the withdrawal response can be measured as the cutaneous nociception threshold, as shown in examples and the like described later.
The cold sensitivity refers to the responsiveness of an animal shown when a cold stimulus is applied. In order to measure the cold sensitivity, it is possible to select and use various known methods according to the type of animals. For example, in a case of a rat, it is possible to measure the cold sensitivity as the frequency of response obtained when acetone is applied to a rear foot of the rat by an acetone method, as shown in examples and the like described later.
The thermosensitivity refers to the responsiveness of an animal shown when a thermal stimulus is applied. In order to measure the thermosensitivity, it is possible to select and use various known methods according to the type of animals. For example, in a case of a rat, it is possible to measure the thermosensitivity as latency of a foot withdrawal response shown when a rear foot of a rat is stimulated with heat of infrared by the method of Hargreaves et al, as shown in examples and the like described later.
Meanwhile, depression can be evaluated using the increase in the immobility time in a forced swimming test as an index. However, the measurement of depression is not limited to this index, and another index, for example, the immobility time at the time of tail suspension, spontaneous motor activity, or the like can also be used.
The therapeutic effect of 5-HT_{2C} receptor agonist on fibromyalgia can be confirmed by an experiment using other appropriate disease animal models and can also be confirmed by a clinical test on patients with fibromyalgia.

Although producing sufficient effects even when being administered singly, the medicinal agent of the present invention can be concurrently used with an anxiolytic (flurazepam or the like), an antidepressant (duloxetine, milnacipran, amitriptyline, fluvoxamine, or the like), an anticonvulsant (pregabalin, gabapentin, or the like), a sleep improving drug (sodium oxybate, ramelteon, melatonin, or the like), or with a general analgesic (morphine, tramadol, diclofenac, naproxen, acetaminophene, or the like), or the like, which are used for alleviating the symptoms of patients with fibromyalgia, at the same time or at a time interval.

### Examples

Hereinbelow, the present invention will be described in more detail based on examples and the like, but the present invention is not limited to the examples and the like.

### Reference Example 1: Preparing Disease Animal Model of Fibromyalgia Induced by Repeated Reserpine Administration (Lowering of Muscle Tenderness Threshold and Cutaneous Nociception Threshold)

### 1. Experimental Method

Male and female Sprague-Dawley rats (Japan SLC, Hamamatsu, Japan) were used.
The muscle tenderness threshold was measured according to the method of Schafers et al (Schafers M et al,. Pain, 104, 579-588, 2003). A pressure stimulus gradually increasing up to 250 g was applied to the gastrocnemius of the right rear foot of the rat. The magnitude of a minimum pressure stimulus at which the rat showed a withdrawal response with respect to the pressure stimulus of the right rear foot was measured as a muscle tenderness threshold (g). The measurement was performed for three times for each point in time, and the average thereof was taken as a measured value. The cutaneous nociception threshold was measured according to the method of Chaplan et al (Chaplan et al., J Neurosci Methods, 53, 55-63, 1994). The measurement was performed using a series of von Frey filaments differing in thickness, which made it possible to apply a constant pressure. The rats were adapted to a cage for measurement having a wire mesh bottom, and then von Frey filaments (0.42 g, 0.74 g, 1.2 g, 2.1 g, 3.5 g, 6.0 g, 9.3 g, and 15.8 g) were applied to the plantar surface of a right rear foot of the rat until the withdrawal response was caused in the right rear foot or until 6 seconds elapsed without the response. The von Frey filament was applied using an up and down method. That is, a filament having about a medium thickness was applied initially, and when the withdrawal response was observed, a filament having a thickness which is one level lower than the initial filament was applied, and when the response was not observed, a filament having a thickness which is one level higher than the initial filament was applied. The cutaneous nociception threshold at the respective points in time was calculated as a threshold (g) of a foot withdrawal response by using a calculation formula reported by Chaplan et al (Chaplan et al. described above). The influence of reserpine on the muscle tenderness threshold and the threshold of cutaneous nociception was examined according to the following protocol. 24 rats were used for male and female rats respectively. The muscle tenderness threshold and the cutaneous nociception threshold before the reserpine administration (base line) were measured, and then the rats were assigned to any of a vehicle (0.5% aqueous acetic acid) group, a 0.1 mg/kg reserpine group, a 0.3 mg/kg group, and a 1 mg/kg group based on the dose of reserpine (6 rats per group). A vehicle or reserpine was subcutaneously administered to the back of rats of the respective groups once a day for three days. The volume of the vehicle or reserpine was set to 1 ml per a weight of a rat of 1 kg for all groups. On the first, third, fifth, seventh, tenth, fourteenth, and twenty-first days after the final administration of the vehicle or reserpine, the muscle tenderness threshold and the cutaneous nociception threshold were measured for the respective rats. The muscle tenderness threshold and the cutaneous nociception threshold were measured by an experimenter who did not know the dose of reserpine administered to the animal.
All measured values were indicated as an average±standard error. The statistical significant difference in the measured value at the respective points in time between the reserpine-administered group and the vehicle-administered group was verified by two way ANOVA and a Bonferroni method. A significance level (P) less than 5% was determined as statistical significance (P<0.05 was indicated as * in the drawings).

### 2. Results

### (1) Muscle tenderness threshold

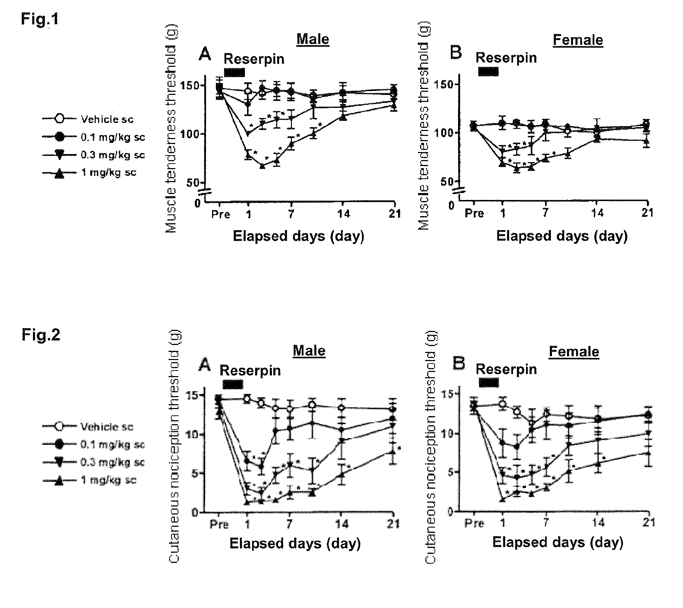

Figs. 1A and 1B respectively show the results in the male and female rats. The reserpine administration significantly lowered the muscle tenderness threshold in all of the male and female rats (two way ANOVA). The muscle tenderness threshold was markedly lowered in the 1 mg/kg reserpine group. Compared to the vehicle group, the muscle tenderness threshold in the 1 mg/kg reserpine group was statistically significantly lower for 10 days after the final administration of reserpine in male rats and for 7 days after the final administration of reserpine in female rats (two way ANOVA and Bonferroni method).

### (2) Cutaneous nociception threshold

Figs. 2A and 2B respectively show the results in the male and female rats. The reserpine administration significantly lowered the cutaneous nociception threshold in all of the male and female rats (two way ANOVA). The cutaneous nociception threshold was markedly lowered in the 1 mg/kg reserpine group. Compared to the vehicle group, the cutaneous nociception threshold in the 1 mg/kg reserpine group was statistically significantly lower for 21 days after the final administration of reserpine in male rats and for 14 days after the final administration of reserpine in female rats (two way ANOVA and Bonferroni method).
From these results, it was confirmed that disease animal models of fibromyalgia were prepared in which the muscle tenderness threshold and the cutaneous nociception threshold were markedly and consistently lowered (tactile allodynia: pain induced by a tactile stimulus generally not causing pain) in the male and female rats by reserpine that was repeatedly subcutaneously administered at 1 mg/kg once a day for three days. In addition, 5 days after the final administration of reserpine, the effect of diclofenac which is a non-steroidal anti-inflammatory drug on the reserpine-induced lowering of the muscle tenderness threshold and the lowering of cutaneous nociception threshold was measured. As a result, the lowering of the muscle tenderness threshold or the lowering of cutaneous nociception threshold was not restored by the diclofenac administered up to a dose of 10 mg/kg. This result was consistent with the findings that ibuprofen (Yunus MB et al., J Rheumatol, 16, 527-532, 1989) or naproxen (Goldenberg DL et al., Arthritis Rheum, 29, 1371-1377, 1986) which is a non-steroidal anti-inflammatory drug is not significantly effective for treating patients with fibromyalgia. Accordingly, it was more clearly confirmed that the disease animal model of fibromyalgia was prepared.

### Reference Example 2: Preparing Disease Animal Model of Fibromyalgia Induced by Repeated Reserpine Administration (Increase in Immobility Time in Forced Swimming Test)

### 1. Experimental method

Male Sprague-Dawley rats (7-week old, Japan SLC, Hamamatsu, Japan) were used as animals. A forced swimming test was performed by modifying the method of Porsolt (1977) et al in the following manner. Soft wires including a magnet (a diameter of 1 mm and a length of 3 mm) were mounted on both the forelimbs of the animal, and the animals were caused to swim one by one in a cylindrical plastic water tank (a diameter of 20 cm and a height of 45 cm) filled with water (24±1°C) up to 30 cm depth. In this experiment, an automatic measurement instrument for forced swimming (MicroAct Scratching Test version 1.06; Neuroscience, Tokyo, Japan) that detects the movement of the magnet-mounted forelimb through a coil provided around the cylinder was used, thereby measuring the immobility time of the respective animals.
The influence of reserpine on the immobility time in the forced swimming test was examined according to the following protocol. 80 rats in total were divided into 5 experiment schedules (measuring the immobility time on the first, third, fifth, seventh, and fourteenth day after the final administration), and the rats were divided into a vehicle (0.5% aqueous acetic acid)-administered group and a 1 mg/kg reserpine-administered group such that each group included 8 rats. As shown in Reference Example 1, the vehicle or reserpine was repeatedly subcutaneously administered at 1 mg/kg once a day for 3 days. Each forced swimming test includes a pretest session (15 minutes of swimming) and a test session (5 minutes of swimming) 24 hours after the pretest session, and the immobility time was measured in the test session. The pretest session was performed on the day of final administration (that is, immediately after the final administration) and on the second, fourth, sixth, and thirteenth days after the final administration of the vehicle or reserpine, and the test session was performed on the first, third, fifth, seventh, and fourteenth days after the final administration of the vehicle or reserpine. The forced swimming test was performed only once for each rat. All of the measured values were indicated as an average±standard error. The statistical significant difference in the measured value at the respective test schedules between the reserpine-administered group and the vehicle-administered group was verified by a Student's t-test. A significance level (P) less than 5% was determined as statistical significance (P<0.05 was indicated as * in the drawings).

### 2. Results

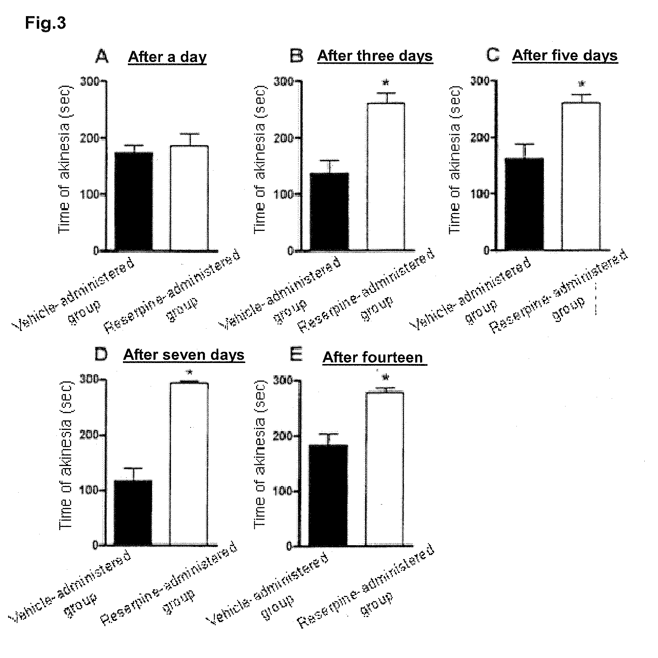

Figs. 3A, 3B, 3C, 3D, and 3E respectively show the results of the forced swimming test performed on the first, third, fifth, seventh, and fourteenth days after the final administration of reserpine. On the first day after the final administration, a significant increase in the immobility time caused by the reserpine administration was not confirmed (P=0.625, Student's t-test). On the other hand, on the third, fifth, seventh, and fourteenth days after the final administration, all of the reserpine-administered group showed a significant increase in the immobility time (P=0.0007, P=0.004, P<0.0001, and P=0.0008 respectively, Student's t-test) compared to the vehicle-administered group. These results show that at least 3 to 14 days after the final administration, depression was induced by reserpine repeatedly administered at 1 mg/kg once a day for 3 days. It is well known that depression is also induced in a high proportion of patients with fibromyalgia, so the present disease animal model has a high similarity to patients with fibromyalgia in respect that the depression is induced as a concomitant symptom.

### Example 1: Effect of 5-HT_{2C} Receptor Agonist on Disease Animal Model of Fibromyalgia

### 1. Experimental method

Male Sprague-Dawley rats (Japan SLC, Hamamatsu, Japan) were used. Thirty rats were used for each drug. As shown in Reference Example 1, reserpine was repeatedly administered at 1 mg/kg once a day for 3 days for 24 rats, thereby causing the lowering of the muscle tenderness threshold and the cutaneous nociception threshold. The other 6 rats were not administered with reserpine and provided for the experiment as a healthy control.
The drug efficacy was evaluated 5 days after the final administration of reserpine for all groups. At this time, the lowering of the muscle tenderness threshold and the cutaneous nociception threshold was marked at a statistically significant level (Reference Example 1), and the immobility time of the forced swimming test, which is an index of depression, was significantly lengthened compared to the control (Reference Example 2).
In evaluating the respective test drugs (5-HT_{2C} receptor agonists), the muscle tenderness threshold of the rats (24 reserpine-administered rats and 6 healthy rats) before the administration of the test drug was initially measured. The 24 reserpine-administered rats were assigned to 4 groups according to the dose of the test drug. For YM348 or a vehicle thereof, the muscle tenderness threshold was measured 30, 60, and 120 minutes after the administration. For lorcaserin or a vehicle thereof, the muscle tenderness threshold was measured 60, 120, and 240 minutes after the administration. For vabicaserin or a vehicle thereof, the muscle tenderness threshold was measured 30, 60, 120, and 240 minutes after the administration. For the 6 healthy rats, only the muscle tenderness threshold was measured without administering the drug. The drug efficacy was measured by an experimenter who did not know how the drug was administered to the animal.
The vehicle, dose, and route of administration of the respective test drugs are shown in the following table.

**[Table 101**

| Drug to be tested | Vehicle | Route of administration | Dose |
|---|---|---|---|
| YM348 | Distilled water | Oral administration | 0, 0.03, 0.1, 0.3 mg/kg |
| Lorcaserin | Distilled water | Oral administration | 0, 0.3, 1, 3 mg/kg |
| Vabicaserin | Physiological saline | Dorsal subcutaneous administration | 0, 0.3, 1, 3 mg/kg |

All measured values were indicated as an average±standard error. The statistical significant difference in the measured value at the respective points in time between the group administered with a test drug and the vehicle-administered group was verified by two way ANOVA and a Bonferroni method. A significance level (P) less than 5% was determined as statistical significance (P<0.05 was indicated as * in the drawings).

### 2. Results

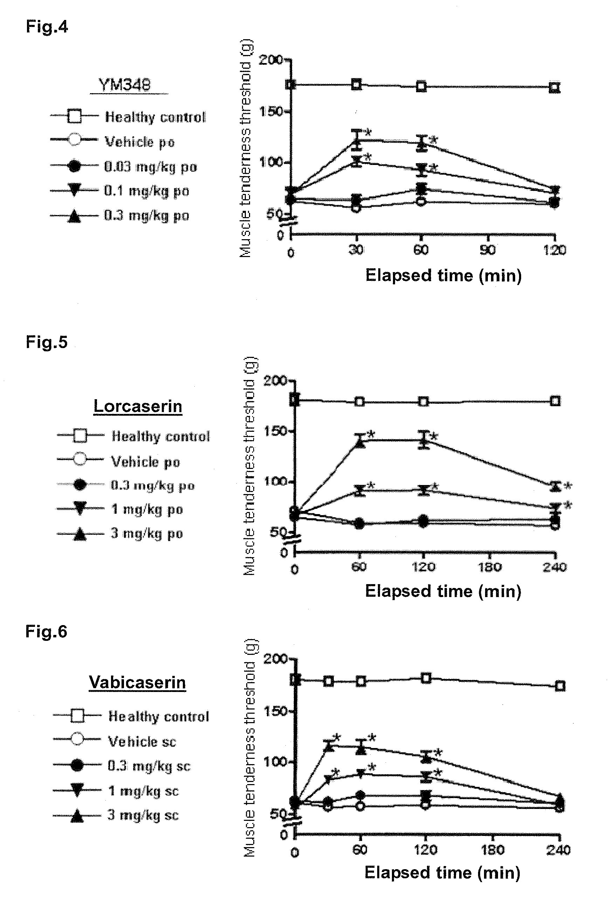

Fig. 4 shows the effect of YM348 on the lowering of the muscle tenderness threshold induced by reserpine. YM348 restored the lowering of the muscle tenderness threshold induced by reserpine in a dose dependent manner. The effect of YM348 was statistically significant at a dose of 0.1 mg/kg or 0.3 mg/kg (two way ANOVA and Bonferroni method).

Fig. 5 shows the effect of lorcaserin on the lowering of the muscle tenderness threshold induced by reserpine. Lorcaserin restored the lowering of the muscle tenderness threshold induced by reserpine in a dose dependent manner. The effect of lorcaserin was statistically significant at a dose of 1 mg/kg or 3 mg/kg (two way ANOVA and Bonferroni method).

Fig. 6 shows the effect of vabicaserin on the lowering of the muscle tenderness threshold induced by reserpine. Vabicaserin restored the lowering of the muscle tenderness threshold induced by reserpine in a dose dependent manner. The effect of vabicaserin was statistically significant at a dose of 1 mg/kg or 3 mg/kg (two way ANOVA and Bonferroni method).

These results showed that a plurality of different 5-HT_{2C} receptor agonists restored the lowering of the muscle tenderness threshold in the disease animal model of fibromyalgia. These results also showed that the 5-HT_{2C} receptor agonist has effects of alleviating chronic pain of deep tissues such as muscle tissues that is a main symptom of patients with fibromyalgia, and alleviating pain caused by a pressure stimulus that is a characteristic symptom of fibromyalgia and used as a diagnosis criterion. That is, these results showed that a medicinal agent containing the 5-HT_{2C} receptor agonist as an active ingredient is useful for treating patients with fibromyalgia.

### Example 2: Antagonism against Action of Compound of Example 1 Caused by 5-HT_{2C} Receptor Antagonist

### 1. Experimental method

Thirty male Sprague-Dawley rats (Japan SLC) were used. As shown in Example 1, reserpine was repeatedly subcutaneously administered at 1 mg/kg once a day for 3 days for 24 rats, thereby causing the lowering of the muscle tenderness threshold. The other 6 rats were not administered with reserpine and provided for the experiment as a healthy control. The drug efficacy was evaluated 5 days after the final administration of reserpine.
As drugs, SB242084 as a selective 5-HT_{2C} receptor antagonist and lorcaserin as a 5-HT_{2C} receptor agonist were used. Twenty four reserpine-administered rats were divided into four groups (n=6/group), and the drug was administered to the respective groups in the following manner.
Group 1: 10% β cyclodextrin (vehicle of SB242084)+distilled water (vehicle of lorcaserin)
Group 2: 10% β cyclodextrin+lorcaserin at 3 mg/kg
Group 3: SB242084 at 0.1 mg/kg+lorcaserin at 3 mg/kg
Group 4: SB242084 at 1 mg/kg+lorcaserin at 3 mg/kg
SB242084 or the vehicle thereof was administered by intraperitoneal administration, and lorcaserin or the vehicle thereof was orally administered. In addition, SB242084 or the vehicle thereof was administered 15 minutes before lorcaserin or the vehicle thereof was administered. The muscle tenderness threshold was measured 60 minutes after lorcaserin or the vehicle thereof was administered. For the 6 healthy rats, only the muscle tenderness threshold was measured without administering the drug. The drug efficacy was measured by an experimenter who did not know how the drug was administered to the animal.
All measured values were indicated as an average±standard error. The statistically significant difference in the measured value between the group 1 and the group 2 was verified by a Student's t-test. A significance level (P) less than 5% was determined as statistical significance (P<0.001 was indicated as ### in the drawing). The statistically significant difference in the antagonistic action of SB242084 against the action of lorcaserin was tested by Dunnett's multiple comparison test between groups 2, 3, and 4. At this time, group 2 was used as a comparative control. A significance level (P) less than 5% was determined as statistical significance (P<0.001 was indicated as *** in the drawing).

### 2. Results

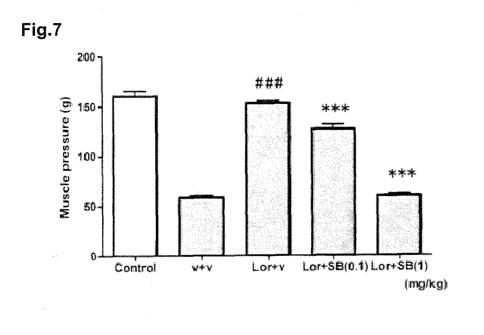

Fig. 7 shows the antagonistic action of SB242084 against the action of lorcaserin. Compared to the vehicle administered group (v+v), lorcaserin (Lor+v) statistically significantly restored the lowering of the muscle tenderness threshold induced by reserpine (Student's t-test). The SB242084 (Lor+SB) showed a statistically significant antagonistic action in a dose dependent manner against the action of lorcaserin (Dunnett's multiple range test).
This result showed that the action of the 5-HT_{2C} receptor agonist of Example 1 restoring the lowering of the muscle tenderness threshold is inhibited by the 5-HT_{2C} receptor antagonist. That is, the result showed that the action of the 5-HT_{2C} receptor agonist of Example 1 restoring the lowering of the muscle tenderness threshold is mediated by the action of the 5-HT_{2C} receptor agonist.

### Industrial Applicability

According to the present invention, it is possible to provide an agent for treating fibromyalgia which is a refractory chronic disease for which a sufficiently effective therapeutic method has not been found.

## Claims

1. An agent for treating fibromyalgia, comprising:
a 5-HT_{2C} receptor agonist as an active ingredient.

2. The treatment agent according to Claim 1,
wherein the active ingredient is a compound represented by the following Formula (I) or a pharmaceutically acceptable salt thereof, 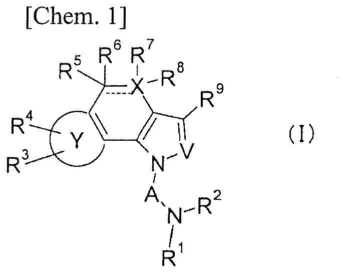 (symbols in the formula mean the following:
ring Y represents an unsaturated 5-membered hetero ring which may have 1 to 3 of one or two or more kinds of hetero atoms selected from N, O and S atoms, or represents an unsaturated 6-membered hetero ring having 1 to 2 N atoms;
X represents a bond or C;
V represents N or CH;
A represents linear or branched C₁₋₆ alkylene which may be substituted with halogen or C₃₋₈ cycloalkyl;
R¹ and R² may be the same as or different from each other and represent H or C₁₋₆ alkyl, alternatively, R¹ and R² or R¹ and A may form a 3- to 8-membered nitrogen-containing saturated hetero ring together with an N atom to which R¹ and R² or R¹ and A bind;
R³ and R⁴ may be the same as or different from each other and represent H, C₁₋₆ alkyl, OH, C₁₋₆ alkyl-O-, amino, C₁₋₆ alkyl-NH-, (C₁₋₆ alkyl)₂-N-, C₁₋₆ alkyl-CO-NH-, or halogen;
R⁵ to R⁹ may be the same as or different from each other and represent H, C₁₋₆ alkyl, OH, or C₁₋₆ alkyl-O-; and
the portion of a dotted line represents an arbitrary double bond;
provided that when the portion of a dotted line is a double bond, R⁶ and R⁸ do not exist, and when X is a bond, the portion of a dotted line is a single bond, and R⁷ and R⁸ do not exist).

3. The treatment agent according to Claim 2,
wherein the active ingredient is a compound represented by the following Formula (I') or a pharmaceutically acceptable salt thereof, 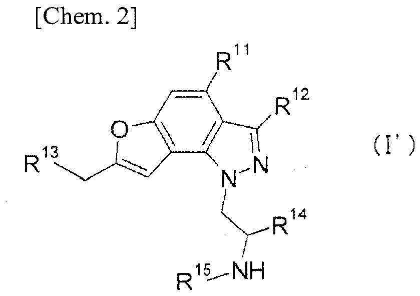 (symbols in the formula mean the following:
R¹¹, R¹², R¹⁴, and R¹⁵ may be the same as or different from each other and represent H or C₁₋₆ alkyl; and
R¹³ represents C₁₋₅ alkyl).

4. The treatment agent according to Claim 3,
wherein the active ingredient is one of the following compounds or a pharmaceutically acceptable salt thereof:
(S)-2-(7-ethyl-1H-furo[2,3-g]indazol-1-yl)-1-methylethylamine;
(S)-2-(7-ethyl-3-methyl-1H-furo[2,3-g]indazol-1-yl)-1-methylethylamine;
(S)-2-(3,7-diethyl-1H-furo[2,3-g]indazol-1-yl)-1-methylethylamine;
(S)-1-methyl-2-(7-propyl-1H-furo[2,3-g]indazol-1-yl)ethylamine;
(S)-N-ethyl-2-(7-ethyl-1H-furo[2,3-g]indazol-1-yl)-methylethylamine;
(S)-2-(4,7-diethyl-1H-furo[2,3-g]indazol-1-yl)-1-methylethylamine;
(S)-2-(7-butyl-1H-furo[2,3-g]indazol-1-yl)-1-methylethylamine; or
(S)-2-(7-isopentyl-1H-furo[2,3-g]indazol-1-yl)-1-methylethylamine.

5. The treatment agent according to Claim 4,
wherein the active ingredient is (S)-2-(7-ethyl-1H-furo[2,3-g]indazol-1-yl)-1-methylethylamine or a pharmaceutically acceptable salt thereof.

6. The treatment agent according to Claim 1,
wherein the active ingredient is a compound represented by the following Formula (II) or a pharmaceutically acceptable salt thereof, 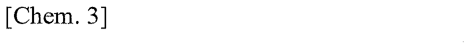 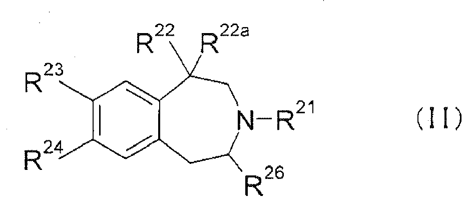 (symbols in the formula mean the following:
R²¹ represents H or C₁₋₈ alkyl;
R²² represents C₁₋₈ alkyl, -CH₂-O-C₁₋₈ alkyl, -C(=O)-O-C₁₋₈ alkyl, -C(=O)-NH-C₁₋₈ alkyl, OH, or CH₂OH;
R^{22a} represents H, alternatively, R²² and R^{22a} form -CH₂-CH₂- together;
R²³ represents halogen, perhalo-C₁₋₈ alkyl, CN, -SR²⁵, -NHR²⁵, -N(R²⁵)₂, aryl, or heteroaryl, wherein the aryl may be optionally substituted with one or two substituents selected from C₁₋₈ alkyl, halogen, perhalo-C₁₋₈ alkyl, and C₁₋₈ alkyl-O-, and the heteroaryl may be optionally substituted with one or two substituents selected from halogen and C₁₋₈ alkyl;
R²⁴ represents H, halogen, perhalo-C₁₋₈ alkyl, CN, -SR²⁵, -NHR²⁵, -N(R²⁵)₂, aryl, or heteroaryl, wherein the aryl may be optionally substituted with one or two substituents selected from C₁₋₈ alkyl, halogen, perhalo-C₁₋₈ alkyl, and C₁₋₈ alkyl-O-, and the heteroaryl may be optionally substituted with one or two substituents selected from halogen and C₁₋₈ alkyl;
alternatively, R²³ and R²⁴ may form a 5- or 6-membered hetero ring having one O atom together with an atom to which R²³ and R²⁴ bind;
R²⁵ independently represents C₁₋₈ alkyl, C₁₋₈ alkenyl, aryl, heteroaryl, aryl-C₁₋₈ alkyl-, heteroaryl-C₁₋₈ alkyl- or perhalo-C₁₋₈ alkyl, or allyl; and
R²⁶ represents H or C₁₋₈ alkyl;
provided that when R²⁶ is C₁₋₈ alkyl, R²⁴ represents a group other than H).

7. The treatment agent according to Claim 6,
wherein the active ingredient is one of the following compounds or a pharmaceutically acceptable salt thereof,
8-chloro-1-methyl-2,3,4,5-tetrahydro-1H-3-benzazepine;
8-bromo-1-methyl-2,3,4,5-tetrahydro-1H-3-benzazepine;
8-iodo-1-methyl-2,3,4,5-tetrahydro-1H-3-benzazepine;
8-trifluoromethyl-1-methyl-2,3,4,5-tetrahydro-1H-3-benzazepine;
8-trifluoromethyl-1-ethyl-2,3,4,5-tetrahydro-1H-3-benzazepine;
8-chloro-1-ethyl-2,3,4,5-tetrahydro-1H-3-benzazepine;
8-bromo-1-ethyl-2,3,4,5-tetrahydro-1H-3-benzazepine;
8-iodo-1-ethyl-2,3,4,5-tetrahydro-1H-3-benzazepine;
7,8-dichloro-1-methyl-2,3,4,5-tetrahydro-1H-3-benzazepine;
7,8-dichloro-1-ethyl-2,3,4,5-tetrahydro-1H-3-benzazepine
8-chloro-7-fluoro-1-methyl-2,3,4,5-tetrahydro-1H-3-benzazepine; or 8-chloro-7-fluoro-1-ethyl-2,3,4,5-tetrahydro-1H-3-benzazepine.

8. The treatment agent according to Claim 7,
wherein the active ingredient is (R)-8-chloro-1-methyl-2,3,4,5-tetrahydro-1H-3-benzazepine or a pharmaceutically acceptable salt thereof.

9. The treatment agent according to Claim 1,
wherein the active ingredient is a compound represented by the following Formula (III) or a pharmaceutically acceptable salt thereof, 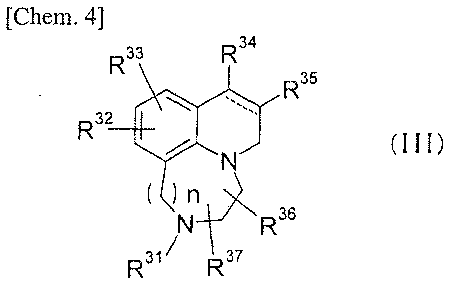 (in the formula,
R³¹ represents H, C₁₋₆ alkyl, C₁₋₅ alkyl-C(=O)-, or phenyl-C₁₋₆ alkyl-O-C(=O)-;
each of R³² and R³³ independently represents H, OH, C₁₋₆ alkyl, C₁₋₆ alkyl-O-, halogen, NH₂-C(=O)-, C₁₋₅ alkyl-O-C(=O)-, perfluoro-C₁₋₆ alkyl, cyano, C₁₋₆ alkyl-S(O)₂-NH-, C₁₋₆ alkyl-S(O)₂-, C₁₋₅ alkyl-C(=O)-NH-, amino, C₁₋₆ alkyl-NH-, (C₁₋₆ alkyl)₂-N-, perfluoro-C₁₋₆ alkyl-O-, C₁₋₅ alkyl-C(=O)-O-, C₁₋₅ alkyl-C(=O)-, phenyl-C(=O)-, phenyl, phenyl-C₁₋₆ alkyl-, heteroaryl, or heteroaryl-C₁₋₆ alkyl-, wherein a phenyl or heteroaryl portion of all substituents having the phenyl or heteroaryl portion may be substituted with 1 to 3 substituents independently selected from halogen, C₁₋₆ alkyl, or C₁₋₆ alkyl-O-;
each of R³⁴ and R³⁵ independently represents H or C₁₋₆ alkyl, alternatively, R³⁴ and R³⁵ may form a cyclic portion selected from monocyclic C₄₋₈ alkane, monocyclic C₄₋₈ alkene, bridged bicyclic C₅₋₁₀ alkane, bridged bicyclic C₅₋₁₀ alkene, pyran, and thiopyran (wherein, a S atom may be oxidized to SO or SO₂) together with carbon to which R³⁴ and R³⁵ bind, wherein the cyclic portion formed by R³⁴ and R³⁵ may be substituted with 1 to 3 substituents selected from halogen, C₁₋₆ alkyl, and C₁₋₆ alkyl-O-;
each of R³⁶ and R³⁷ independently represents H or C₁₋₆ alkyl; and
n represents 1 or 2;
provided that, the portion of a dotted line represents an arbitrary double bond;
wherein, heteroaryl means a 5- to 7-membered monocyclic aromatic ring group having 1 or 2 hetero atoms selected from N, O, and S).

10. The treatment agent according to Claim 9,
wherein the active ingredient is one of the following compounds or a pharmaceutically acceptable salt thereof,
4,5,6,7,9,9a,10,11,12,12a-decahydrocyclopenta[c][1,4]diazepino[6,7,1-ij]quinoline;
2-bromo-4,5,6,7,9,9a,10,11,12,12a-decahydrocyclopenta[c][1,4]diazepino[6,7,1 - ij]quinoline;
2-chloro-4,5,6,7,9,9a,10,11,12,12a-decahydrocyclopenta[c][1,4]diazepino[6,7,1 - ij]quinoline;
2-phenyl-4,5,6,7,9,9a,10,11,12,12a-decahydrocyclopenta[c][1,4]diazepino[6,7,1-ij]quinoline;
2-methoxy-4,5,6,7,9,9a,10,11,12,12a-decahydrocyclopenta[c][1,4]diazepino[6,7,1-ij]quinoline;
1-fluoro-4,5,6,7,9,9a,10,11,12,12a-decahydrocyclopenta[c][1,4]diazepino[6,7,1 - ij]quinoline;
1-(trifluoromethyl)-4,5,6,7,9,9a,10,11,12,12a-decahydrocyclopenta[c][1,4]diazepino[6,7,1-ij]quinoline; or
1-fluoro-2-methoxy-4,5,6,7,9,9a,10,11,12,12a-decahydrocyclopenta[c][1,4]diazepino[6,7,1-ij]quinoline.

11. The treatment agent according to Claim 10,
wherein the active ingredient is (-)-(9aR, 12aS)-4,5,6,7,9,9a,10,11,12,12a-decahydrocyclopenta[c][1,4]diazepino[6,7,1-ij]quinoline or a pharmaceutically acceptable salt thereof.

12. The treatment agent according to Claim 1,
wherein the active ingredient is
(S)-2-(7-ethyl-1H-furo[2,3-g]indazol-1-yl)-1-methylethylamine;
(1R)-8-chloro-1-methyl-2,3,4,5-tetrahydro-1H-3-benzazepine;
(-)-(9aR, 12aS)-4,5,6,7,9,9a,10,11,12,12a-decahydrocyclopenta[c][1,4]diazepino[6,7,1-ij]quinoline;
or a pharmaceutically acceptable salt thereof.
